# EUROPÄISCHE PATENTANMELDUNG

(11) **EP 2 292 791 A1**
(43) Veröffentlichungstag der Anmeldung: **09.03.2011**
(21) Anmeldenummer: 10186096.3
(22) Anmeldetag: 30.10.2001
(51) Int. Cl.: C12Q 1/68, C12Q 1/70, C12N 5/10, C12N 15/10, C12N 15/62

(54) **Kernexportreportersystem**

(30) Priorität: 30.10.2000 DE 10053781
(62) Teilanmeldung aus: 08013449.7
(71) Anmelder: GENEART AG, 93053 Regensburg (DE)
(72) Erfinder: Wagner, Ralf, 93049 Regensburg (DE); Graf, Marcus, 93053 Regensburg (DE)
(74) Vertreter: Weiss, Wolfgang

(57) **Zusammenfassung**

Die vorliegende Erfindung betrifft Reportersysteme für den RNA-Export, Verfahren zur Suche nach Molekülen, die den RNA-Export beeinflussen und eine auf diesen Verfahren beruhende Methode zum Nachweis einer Virusinfektion.

## Beschreibung

Die vorliegende Erfindung betrifft Reportersysteme, die es erlauben, Proteine, Signalsequenzen und/oder Wirkstoffkandidaten zu identifizieren, die den Export von RNAs (ribonucleic acids) und insbesondere viralen RNAs aus dem Zellkern eukaryontischer Zeilen regulieren,

Verschiedenste Viren sind auf einen aktiven Export ihrer unvollständig gespleißten Transkripte aus dem Zellkern der infizierten Wirtszelle angewiesen. Dies kann entweder über die Verwendung eines cis-ständigen RNA Signales innerhalb der viralen Transkripte (konstitutive Transportelemente) erfolgen, oder geschieht mit Hilfe viraler Proteine. Cis-aktive Transportelemente werden beispielsweise von MPMV-CTE (Mason-Pfizer monkey Virus constitutive transport element), SRV-CTE (simian retrovirus constitutive transport element), Hepatitis B Virus PRE (posttranscriptional regulatory element) und HSV (herpes simplex virus) (innerhalb des TK (Thymidinkinase)-Gens) verwendet. Diese RNA-Elemente rekrutieren zelluläre Faktoren und Exportwege, um den Kernexport der viralen Transkripte zu ermöglichen, Alternativ dazu kann der Kernexport auch über einen Exportfaktor vermittelt werden, der spezifisch an eine Zielsequenz innerhalb der viralen Transkripte bindet und diese im Zusammenspiel mit zellulären Faktoren in das Cytoplasma transportiert. So werden beispielsweise Ad-5 Transkripte mit Hilfe der 34K und E4orf6 Proteine, EBV (Epstein-Barr virus) Transkripte mit Hilfe des EB2 Proteins, Herpesvirus Saimiri Transkripte mit Hilfe des ORF 57 Genproduktes, HSV (herpes simplex virus) Transkripte mit Hilfe des ICP 27 Proteins, HTLV-1 und 11 (human T-cell leukemia virus 1 and ll) Transkripte mit Hilfe der Rex-Proteine, EIAV (equine infectious anemia virus), SIV (simian immunodefidency virus), HIV-1 und HIV-2 (human immunodefidency virus 1 and 2) Transkripte mit Hilfe-, der Rev exportiert.

Am besten untersucht ist der HIV-1 Rev vermittelte Kernexport später HIV-1 Transkripte. Wie alle Lentiviren ist HIV-1 darauf angewiesen, aus nur einer proviralen Matrize mehrere Gene zu aktivieren und in einer zeitlich festgelegten Abfolge zu exprimieren. Durch alternative Spleißereignisse sowie weiteren, auf RNA Ebene stattfindenden Regulationsmechanismen, werden unterschiedliche Gene aus nur einem -9kB großen Primärtranskript generiert. Diese viralen Transkripte können aufgrund ihrer Größe in 3 Klassen aufgeteilt werden: -9kB ungespleißte (gag, pol), -4kB einfach gespleißte (env, vif, vpr, vpu) und -2kB mehrfach gespleißte (rev, tat, nef) RNAs.

Neben dem Auftreten von unvollständig bis mehrfach gespleißten Transkripten kann zudem eine zeitliche Abfolge in der Expression dieser unterschiedlichen RNA Spezies beobachtet werden. So sind in der frühen Phase der Replikation im Cytoplasma der infizierten Zellen nur die mehrfach gespleißten -2 kB RNAs, und ihre Genprodukte Rev, Tat und Nef nachweisbar. Erst zeitlich verzögert treten dort dann auch die un- (-9 kB) und einfach (-4 kB) gespleißten Transkripte und ihre Genprodukte Gag, Pol und Env in Erscheinung. In Zellen, die mit Virus-Mutanten, denen ein aktives Rev Protein fehlt, infiziert wurden, können die einfach- und nichtgespleißten Transkripte jedoch nie im Cytoplasma nachgewiesen werden. Die un- und einfach gespleißten Transkripte akkumulieren dann im Kern (siehe Abb. A-4), und die von ihnen translatierten späten Strukturproteine (Gag, Env) und Enzyme (Pol) können nicht gebildet werden. Das virale Rev Protein ist also in essentieller Weise an der zeitlich regulierten Expression der viralen Gene beteiligt.

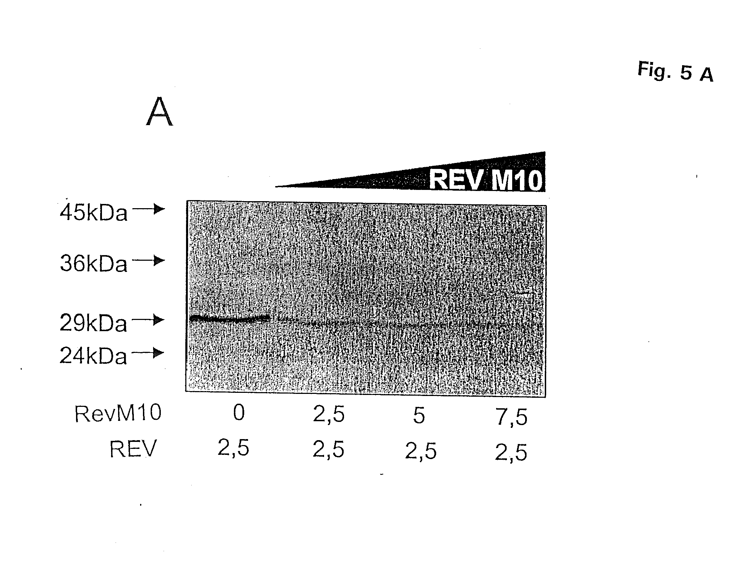

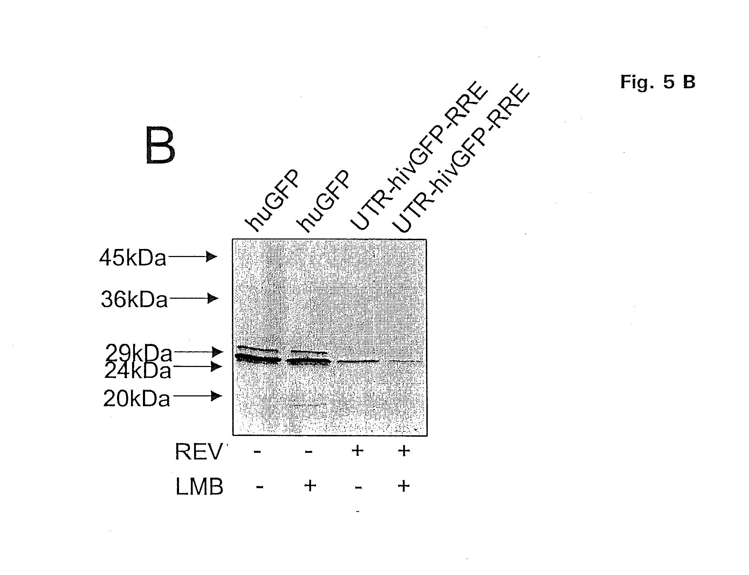

HIV-1 Rev, ebenso wie die oben aufgeführten RNA Transportmoleküle sind Shuttle-proteine, die virale RNAs über die Wechselwirkung mit einer innerhalb viraler Transkripte gelegenen RNA Zielsequenz diese aus dem Kern, in das Cytoplasma transportieren. So bindet HIV-1 Rev im Kern spezifisch an seine RNA Zielstruktur RRE, das "Rev-responsive-element" (siehe Abb. 5 A/B). Diese 351 Nukleotide (Nt) lange Region ist innerhalb des Env Leserahmens lokalisiert und damit Bestandteil aller un- und einfach gespleißten Transkripte. Anschließend wird dieser Ribonukleoprotein(RNP)-Komplex über die Interaktion mit zellulären Faktoren aus dem Zellkern exportiert. Dazu ist eine C-terminal gelegene Leucin-reiche Sequenz notwendig, die als Kernexportsequenz NES ("nuclear export sequence") die nukleäre Translokation des Rev Proteins unter Verwendung zellulärer Mechanismen vermittelt (Pollard and Malim, 1998).

Immer noch umstritten ist der Grund, warum die späten Transkripte in Abwesenheit von Rev im Kern zurückbleiben, was eine notwendige Voraussetzung für die Rev-Abhängigkeit und damit die zeitlich regulierte Expression von Gag, Pol und Env ist. Im Prinzip dominieren zwei alternative Vorstellungen zur Kernretention später Transkripte.

Es wird angenommen, daß ein zelluläres Transkript den Zellkern erst dann verlassen kann, wenn der Spleißprozess vollständig abgeschlossen ist, respektive alle aktiven Spleißstellen aus dem Primärtranskript entfernt worden sind. Bei den späten viralen Transkripten handelt es sich um Intron-haltige, nur unvollständig gespleißte pre-mRNAs, die mit Hilfe von Rev und RRE ins Cytoplasma transportiert werden. Deshalb wurde schon frühzeitig der Einfluß der zellulären Speißmaschinerie auf die Kernretention der späten Transkripte untersucht (Mikaelian et al., 1996; Kjems et al., 1991; Kjems and Sharp, 1993; Chang and Sharp, 1989; Powell et al., 1997; Lu et al., 1990; 0'Reilly et al., 1995). Durch das Vorhandensein unterschiedlich aktiver Spleißstellen scheint der Spleißprozeß bei HIV-1-Transkripten nur suboptimal erfolgen. Deshalb wurde von mehreren Gruppen die Hypothese aufgestellt, daß Rev den Export von Transkripten ermöglicht, die innerhalb der Spleißmaschinerie durch Ausbildung ineffizienter Spleißkomplexe festgehalten werden.

Konträr dazu konnte aber gezeigt werden, daß die späten HIV-1 Gene wie beispielsweise Env auch in Abwesenheit von aktiven Spleißstellen in ihrer Expression reprimiert bleiben und damit der Einfluß der Spleißmaschinerie mehr indirekt zu sein scheint (Nasioulas et al., 1994). Daher wurden sogenannte inhibitorische Sequenzen (INS) oder cis-aktive Repressor Elemente (CRS) innerhalb der Leserahmen postuliert, welche die Expression negativ beeinflussen (Nasioulas et al., 1994; Olsen et al., 1992; Schwartz et al., 1992b; Maldarelli et al., 1991). Diese innerhalb der kodierenden mRNA gelegenen Repressor-Sequenzen besitzen jedoch kein gemeinsames Sequenzmotiv wie etwa das AUUUA-Instabilitätsmotiv innerhalb des 3'-UTR der instabilen GM-CSF mRNA (Chen and Shyu, 1995), sondern fallen nur durch ihren durchweg hohen A/U Gehalt auf. So resultierte die Fusion der postuüerten INS-haltigen Fragmente aus Leserahmen später Gene (wie Gag und Env) an ein CAT-Reportersystem in eine reduzierte Reporteraktivität (Cochrane et al., 1991; Rosen et al., 1988; Schwartz et al., 1992b). Diese Reduktion der Expression von Gag und Pol konnte teilweise durch mehrfache stille Punktmutationen innerhalb der "wobble"-Positionen teilweise wieder aufgehoben werden (Schwartz et al., 1992a; Schneider et al., 1997). Die un- und einfach gespleißten HIV-1 mRNAs scheinen also cis-aktive Repressor-Elemente zu besitzen, die entweder durch multiples Spleißen entfernt oder durch einen Rev/RRE vermittelten Kernexport überwunden werden.

An Molekülen, die den Export von RNA, insbesondere viraler RNA, aus dem Zellkern modulieren, insbesondere hemmen, besteht ein großes medizinisches Interesse. Beispielsweise ist HIV-1 Rev ein essentieller Faktor während der Hi-viralen Replikation und dies sowohl in Zellkultur als auch in vivo. (Feinberg, Jarrett, 1986; Iversen, Shpaer, 1995; Sodroski, Goh, 1986). Dies macht Rev zu einem attraktiven Ziel für antiviral wirksame Therapeutika.

Rev-sensitive Reportersysteme können zur Austestung von Wirkstoffen verwandt werden, die eine Rev-Funktion unterbinden, und somit eine produktive Replikation von HIV-1 verhindern. Zur Evaluierung der Rev-abhängigen HIV-1 Genexpression wurden bislang verschiedenste Systeme angewandt. Diese reichen von mutierten Provirus-Konstrukten (Borg et al., 1997; Malim and Cullen, 1993) über chimäre Rev-sensitive ß-Globin Gene (Chang and Sharp, 1989; Mikaelian et al., 1996) bis hin zu subgenetischen Fragmenten, die an Reportergene fusioniert wurden (Schwartz et al., 1992a; Schwartz et al., 1992b).

Die bislang verwendeten Systeme eigen sich jedoch nicht zur Hochdurchsatztestung (HDT) von Rev-inhibierend wirkenden Therapeutika, oder zur Isolierung von spezifischen Inhibitoren der Rev-Funktion aus einer randomisierten Genbank: Die Arbeit mit replikationsfähigen viralen Systemen oder Proviruskonstrukten erfordert S3 Sicherheitslabors, sowie aufwendige und teure (p24 capture ELISA) Nachweismethoden, die eine HDT per se nicht zulassen oder unattraktiv machen. Komplexe Rev-abhängige ß-Globin Reportersysteme eignen sich nur für die Grundlagenforschung, und erfordern spezielle, anspruchsvolle Labormethoden (Northern Blot, RNA protection assay), die ebenfalls eine HDT ausschließen. Reportersysteme, die auf einer speziellen enzymatischen Reaktion zum Nachweis einer Rev-sensitiven Genexpression basieren, erlauben wenigstens z.T. eine HDT. So konnte die Expression des Chloramphenicol Acetyltransferase (CAT) Gens in die Rev-Abhängigkeit überführt werden, indem der CAT Leserahmens, zusammen mit der RRE Region innerhalb einer Intronsequenz plaziert wurde (Luo et al., 1994; lacampo, Cochrane, 1996; Hope, Huang, 1990). Dieses Reporterkonstrukt (pDM128 und Derivate) besitzt jedoch eine Reihe von Nachteilen:
- (a): Der Assay kann nicht auf Einzelzellbasis angewandt werden. So können Zellen nicht durch FACS separiert werden, sondern müssen obligat zur Analyse lysiert werden.
- (b): Eine einfache, leicht automatisierbare, optische Austestung einer Rev-Aktion ist nicht möglich.
- (c): Im Cytoplasma von mit pDM128 transfizierten Zellen können auch in Abwesenheit von Rev noch ungespleißte CAT RNAs nachgewiesen werden, d.h. in dem Assay wird auch in Abwesenheit von Rev fälschlich eine Rev-Abhängigkeit vorgetäuscht.
- (d): Für den Assay ist das Vorhandensein einer intakten zellulären Spleißmaschinerie Voraussetzung. Moleküle, die Spleißvorgänge beeinflussen, werden im Assay fälschlich als Modulatoren des RNA-Exports eingestuft.

Aufgabe der vorliegenden Erfindung war es daher, ein Verfahren zum Nachweis des RNA-Exports aus dem Zellkern einer eukaryontischen Zelle bereitzustellen, das die oben genannten Nachteile des Standes der Technik nicht aufweist.

Diese Aufgabe wird gelöst durch ein Verfahren, umfassend die Schritte:
- (a): Bereitstellen einer Nukleinsäure, die für ein Reporterprotein kodiert und deren Transkript abhängig vom Vorhandensein
(i) eines cis-aktiven RNA-Exportsignals in operativer Verknüpfung mit der Nukleinsäure und
(ii) eines trans-aktiven Faktors und gegebenenfalls
(iii) eines funktionalen 5'-Spleißdonors in Abwesenheit eines funktionalen 3'-Spleißakzeptors aus dem Zellkern exportiert wird,
- (b): Einbringen der Nukleinsäure in den Zellkern der Zielzelle, so dass sie dort in operativer Verknüpfung mit einer Transkriptionskontrollsequenz vorliegt, und dass bei Transkription der Nukleinsäure ein Transkript erzeugt wird, bei dem der für das Reporterprotein kodierende Abschnitt des Transkripts keinem Spleißvorgang unterzogen werden kann,
- (c): Transkribieren der Nukleinsäure und
- (d): Bestimmen, ob das resultierende Transkript aus dem Zellkern exportiert wird.

Die Anwesenheit des 5'-Spleißdonors darf nicht dazu führen, dass wie bei den Konstrukten von Lu et al. ein Spleißereignis möglich wird. Entsprechend darf dem 5'-Spleißdonor kein 3'-Spleißakzeptor oder zumindest kein effizient genutzter Spleißakzeptor gegenüber stehen.

Das cis-aktive RNA-Exportsignal kann ein bereits bekanntes RNA-Exportsignal sein. In diesem Fall positioniert man das RNA-Exportsignal zum Reportergen bevorzugt analog zum natürlichen Auftreten des Exportsignals. Handelt es sich also beispielsweise um das Rev-responsive element von HIV-1, wird das Signal stromabwärts des Reportergens kloniert. Umgekehrt werden die Exportsignale von Adenoviren bevorzugt stromaufwärts des Reportergens angeordnet.

Das erfindungsgemäße Verfahren eignet sich zur Identifizierung unbekannter RNA-Exportsignale. Dazu wird in der Nukleinsäure das Reportergen operativ verknüpft mit einer Bibliothek von Genfragmenten und nach Konstrukten gesucht, die den RNA-Export ermöglichen. Bevorzugt enthält die Genfragmentbibliothek Gene oder Genfragmente von Spenderorganismen oder Viren, von denen bekannt ist, dass sie über effiziente RNA-Exportsignale verfügen. Falls speziell nach RNA-Exportsignalen gesucht werden soll, die die Anwesenheit viraler in trans wirkender Faktoren erfordern, müssen diese in der Zelle ebenfalls bereitgestellt werden.

Umgekehrt kann, falls ein cis-aktives Exportsignal bekannt ist und ein in trans aktiver Faktor identifiziert werden soll, mit einem Konstrukt wie oben beschrieben nach einem solchen Faktor gesucht werden. In diesem Fall enthält das Nukleinsäurekonstrukt sowohl das Reportergen als auch das cis-aktive Signal. Mit dem oben beschriebenen Reportersystem kann dann in Expressionsbibliotheken nach in trans aktiven Faktoren gesucht werden.

Bei den in trans aktiven Faktoren handelt es sich bevorzugt um virale Adaptermoleküle, die eine Wechselwirkung mit der zellulären RNA-Exportmaschinerie vermitteln, es kann sich aber auch um zelluläre Moleküle handeln. Unter einem solchen in trans aktiven Faktor wird im Sinne dieser Anmeldung sowohl ein einzelnes Protein als auch ein Proteinkomplex verstanden.

Werden alle für den RNA-Export erforderlichen, in cis und trans wirkenden Faktoren zur Verfügung gestellt, eignet sich das System zur Identifizierung von Molekülen, die den RNA-Export beeinflussen. Bei dem Molekül, das den RNA-Export beeinflußt, kann es sich beispielsweise
- (a): um ein kleines Molekül, typischerweise mit einer Molekularmasse kleiner 2000 Da,
- (b): um DNA oder RNA, Derivate oder Mimetika davon handeln, die auf Nukleinsäureebene wirken oder mit Proteinen interagieren, die am RNA-Export beteiligt sind, oder
- (c): um ein Peptid, ein modifiziertes Peptid, Protein oder modifiziertes Protein handeln, das mit Nukleinsäuren wechselwirkt oder mit Proteinen interagiert, die am RNA-Export beteiligt sind, handeln.

Ein Beispiel für ein kleines Molekül nach (a) ist Leptomycin B, ein bekannter Inhibitor der Rev-Aktivität. Ein Beispiel für eine Nukleinsäure nach (b) sind die bekannten RRE-decoys. Ein Beispiel für ein Protein nach (c) ist die transdominant-negative Rev-Mutante RevM10. Insbesondere können die Nukleinsäuren aus einer Genbibliothek stammen oder die Proteine Genprodukte der Gene einer Genbibliothek sein. In diesen Fällen kommt der Vorteil des erfindungsgemäßen Systems, ohne Lyse oder Fixierung der Zellen auszukommen, ganz besonders zum Tragen.

Die Wirksamkeit von Leptomycin B und der transdominant-negativen Rev-Mutante RevM10 konnte mit dem erfindungsgemäßen Reportersystem (mit hivGFP als Reporterprotein) nachgewiesen werden. Das hergestellte Rev-sensitive Reportersystem eignet sich also zur Detektion einer Inhibition der viralen Genxpression und somit als Reportersystem zur Identifizierung anti-viral wirksamer Therapeutika, die auf den viralen Kernexport einwirken.

Eine weitere wesentliche Verbesserung des erfindungsgemäßen Reportersystems gegenüber dem bereits bekannten CAT-Systems besteht in der Vermeidung von Spleißvorgängen. Beim CAT-System wird in jedem Fall RNA in das Cytoplasma exportiert. Nur in Anwesenheit eines in trans aktiven Faktors wie beispielsweise Rev enthält diese noch ein Intron, auf dem das Reportergen kodiert ist. Beim erfindungsgemäßen Reportersystem braucht dagegen kein Spleißereignis zu erfolgen. Die RNA wird in Abwesenheit cis-oder/und trans-aktiver Signale erst gar nicht in Mengen ins Cytoplasma exportiert, die die Anhäufung detektierbarer Mengen RNA erlauben.

Dies kann beispielsweise erreicht werden durch RNA-destabilisierende Sequenzmotive wie beispielsweise AUUUA, das in der RNA für GM-CSF vorkommt, oder bevorzugt durch eine Codonwahl, die die metabolische Stabilität der RNA herabsetzt. Für erfindungsgemäße Reportersysteme geeignete Konstrukte lassen sich beispielsweise erzeugen, indem man die Codonverteilung so wählt, wie sie in viraler exportierter RNA auftritt. Bevorzugt soll hierbei eine Codonwahl verwendet werden, wie in sie Säugerzellen am wenigstens häufig oder am zweitwenigsten häufig verwendet wird (Ausubel et al., 1994), noch mehr bevorzugt wird die Codonwahl an die von späten HIV-1 Genen angepaßt, nochmehr bevorzugt wird Tabelle 1 zur Herstellung des Rev-abhängigen Leserahmens verwendet.

Beispielsweise wurde die Codonwahl des konstitutiv exprimierenden Genes für das grün-fluoreszierende Protein (GFP) an die Codonwahl, wie sie in späten HIV-1 Genen vorzufinden ist, angepaßt. Dazu wurde die Aminosäuresequenz des GFP Genproduktes in einen synthetischen GFP-kodierenden Leserahmen, unter Verwendung der Codonwahl von HIV-1 Gag, rückübersetzt. Dieser hivGFP benannte Leserahmen wurde dann unter Verwendung von langen Oligonukleotiden und einer schrittweisen PCR als vollsynthetischer Leserahmen aufgebaut. Zudem wurde dem hivGFP Leserahmen der authentische 5'-UTR des Gag Leserahmens vorangestellt, sowie das RRE 3' angefügt und in einen Expressionsvektor einkloniert. Der hergestellte hivGFP Vektor, erwies sich in der Expression des autofluoreszenten GFPs als vollständig abhängig von der Präsenz des Rev-Proteins. In Abwesenheit des 5'-UTRs, RRE oder Rev konnte keine Expression des grün leuchtenden Reporters nachgewiesen werden. Das Ausgangs-GFP-Gen, welches in seiner Codonwahl an Säugergene angepaßt war (huGFP), erwies sich in seiner Expression dagegen als unabhängig von Rev, RRE oder dem 5'-UTR.

Statt die Codonwahl möglichst exakt der Codonwahl später HIV-Gene anzupassen, ist es aber auch möglich, lediglich den AT-Gehalt anzuheben. Bevorzugt wird ein AT-Gehalt >50 % angestrebt. Die Erhöhung des AT-Gehalts oder die Anpassung des Codongebrauchs erfolgt vorzugsweise durch stumme Mutationen oder durch Mutationen, die die Aktivität des Reporterproteins nicht zerstören. Die Codonwahl muss nicht angepaßt werden, wenn der A/T-Gehalt des besagten Genes bereits über 50% beträgt. Gene mit vom Wildtyp abweichenden Codongebrauch können wie im Beispiel angegeben zum Beispiel aus langen Oligonukleotiden und mit einer schrittweisen PCR hergestellt werden.

Wegen der besonders einfachen Ausiesbarkeit und der Eignung für Hochdurchsatztests wird als Reporterprotein bevorzugt ein fluoreszierendes Protein verwendet. Beispiele für autofluoreszente Reporterproteine sind das "green-fluorescent-protein" GFP, das "blue-fluorescent-protein" BFP, das "red-fluorescent-protein" RFP, das "yellow-fluorescent-protein" YFP, oder Derivate dieser autofluoreszenten Proteine, die eine erhöhte Fluoreszenz aufweisen wie das "enhanced-green-fluorescent-protein" eGFP, das "enhanced-blue-fluorescent-protein" eBFP, das "enhanced-red-fluorescent-protein" eRFP, oder das "enhanced-yellow-fluorescent-protein" eYFP (Clontech).

Statt mit fluoreszierenden Proteinen zu arbeiten, kann man auch andere Proteine verwenden, sofern deren Aktivität leicht nachweisbar ist. Beispiele sind das Gen für die Luciferase LUC, das Gen für alkalische Phosphatase AP, das Gen für sekretorische alkalische Phosphatase SEAP oder das Gen für die Chloramphenicol Acetyltransferase CAT.

Immunologisch nachweisbare Proteine sind ebenfalls als Reporterproteine geeignet. Es genügt, dass ein schneller immunologischer Nachweis des Genprodukts, von Teilen des Genprodukts oder von Epitopen möglich ist. Häufig verwendet wird beispielsweise der Influenzae Flag-Tag.

Auch Proteine, auf die positiv oder negativ selektiert werden kann, sind als Reporterproteine geeignet. Beipielsweise verhindert das Neomycinresistenzgen einen durch G418 verursachten Translationsblock und damit ein Absterben der Zelle. In HAT (Hypoxanthin, Aminopterin, Thymidin) Medium, in dem die de novo Purin- und Pyrimidinbiosynthese blockiert ist, wird die Aktivität von Thymidinkinase essentiell. Umgekehrt kann auf Thymidinkinase defiziente Zellen durch Propagation der Zellen in Bromdeoxyuridin selektiert werden. Ebenso bewirkt die Enzymaktivität der herpesviralen Thymidinkinase (TK) in Anwesenheit von Acyclovir das Absterben von TK exprimierenden Zellen. Weitere Beispiele für Marker, auf die positiv und negativ selektiert werden kann, sind Adenin-phosphoribosyl-transferase (APRT), Hypoxanthin-guanin-phosphoribosyl-transferase (HGPRT) sowie Dihydrofolatreduktase (DHFR). Auf APRT bzw. HGPRT wird positiv mit Azaserin, negativ mit 8-Azaguanin selektiert. Im Fall der DHFR erfolgt die Selektion auf den Marker mit Methotrexat, die Selektion gegen den Marker mit [³H]dUrd. Alle erwähnten Markersysteme sind ausführlich von Kaufmann beschrieben (Kaufmann, 1979).

Schließlich kann es sich bei dem Reportergen um ein Regulatorgen handeln, das nach seiner Expression in einer Zelle als molekulares Schaltermolekül die Expression andere Gen an- oder abschaltet. Als solches Regulatorgen kann beispielsweise ein Transkriptionsfaktor verwendet werden.

In allen Verfahren, bei denen nicht nach einem cis-aktiven RNA-Exportsignal gesucht werden soll, enthält das Nukleinsäurekonstrukt bereits ein cis-aktives RNA-Exportelement.

Dabei kann es sich kann es sich um ein sogenanntes konstitutives Transportelement handeln. Für den Kernexport von RNAs, die ein solches konstitutives Transportelement tragen, sind keine viralen Proteine für den Export erforderlich, das Virus nützt lediglich bereits vorhandene zelluläre Mechanismen aus. Beipiele für solche cis-aktive RNA-Exportelemente sind MPMV CTE, RSV CTE oder SRV CTE.

Viele virale cis-aktive RNA-Exportelemente sind aber auf virale Adapterproteine angeweisen, die die Interaktion mit der zellulären Exportmaschinerie vermitteln. Im Fall von HIV wird als cis-aktives RNA-Exportelement das RRE (Rev responsive element) verwendet. Dieses Signal wird durch das virale Protein Rev erkannt, das eine Leucin-reiche Sequenz hydrophober Aminosäuren enthält, die als Kernexportsignal (NES, nuclear export signal) wirkt. Rev-Rezeptor beim Kernexport ist Crm1, das auch als Exportin 1 bezeichnet wird. Die Interaktion des Rev-Rezeptors mit Crm1 kann durch Leptomycin B gestört werden. Man glaubt, dass das Rex/RxRRE System von HTLV-1 und HTLV-II analog zum Rev/RRE System von HIV-1, HIV-2 und SIV funktioniert.

Für die weiter oben beschriebenen RNA-Exportsysteme befindet sich das RNA-Exportsignal bevorzugt stromabwärts der Strukturgene. Es können aber auch RNA-Exportsignale von Viren verwendet werden, bei denen die RNA-Exportsignale stromaufwärts der Strukturgene liegen, wie dies zum Beispiel bei Adenoviren vorkommt. In diesem Fall befindet sich dann auch bei den RNA-Konstrukten die Kernexportsequenz bevorzugt stromaufwärts des Reportergens.

Ein weiterer Gegenstand der Erfindung ist eine DNA-Sequenz, die für ein Reporterprotein kodiert und in operativer Verknüpfung mit einem cis-aktiven RNA-Exportelement und gegebenenfalls mit einem funktionalen 5'-Spleißdonor in Abwesenheit eines funktionalen 3'-Spleißakzeptors vorliegt, wobei
- (a): die für das Reporterprotein kodierende DNA-Sequenz gegenüber der Wildtypsequenz auf Nukieins"ureebene modifiziert ist,
- (b): für die modifizierte DNA-Sequenz ein RNA-Export in Abhängigkeit vom cis-aktiven RNA-Exportelement erfolgt und
- (c): für die Wildtyp DNA-Sequenz im wesentlichen kein RNA-Export in Abhängigkeit des cis-aktiven RNA-Exportelements erfolgt.

Unter dem Wildtyp wird dabei entweder der Wildtyp im üblichen Sinne oder aber ein für die Expression in der entsprechenden Wirtszelle optimiertes Gen wie beispielsweise "humanisiertes GFP" verstanden. Die Abhängigkeit der modifizierten Reporter-RNA vom cis-aktiven RNA-Exportelement wird mit den oben beschriebenen Methoden erreicht.

Die Transkription der entsprechenden RNA-Sequenz wird durch eine Transkriptionskontrollsequenz kontrolliert. Die Transkriptionskontrollsequenz kann einen konstitutiven oder induzierbaren Promotor enthalten. Als konstitutive Promotoren können beispielsweise virale Promotoren von CMV, SV 40 oder von Adenovirus oder zelluläre Promotoren wie der Aktinpromotor oder zelltypspezifische Promotoren wie der MHCII-Promotor verwendet werden. Als induzierbare Promotoren kommen von Tetracyclin abhängige Promotoren (Tet-On/Off-System), Hitzeschockpromotoren, Metallothioneinpromotoren oder durch Glucocorticoide induzierbare Promotoren wie der Promotor in mouse mammary tumor virus (MMTV) LTR in Frage (Kaufmann, 1979). Zur Polyadenyllerung der RNA-Transkripte wird ein entsprechendes Polyadenylierungssignal an das 3'-Ende der DNA angehängt. Für die Wahl des Reporterproteins und des cis-aktiven RNA-Exportsignals gelten die oben gemachten Ausführungen. Unter Umständen bietet es sich an, einen transaktiven Faktor und das Reporterkonstrukt auf demselben Plasmid vorzusehen.

Ein weiterer Gegenstand der vorliegenden Erfindung sind eukaryontische Zellen, stärker bevorzugt Säugetierzellen, am meisten bevorzugt humane Zellen, die mit einem DNA-Kontrukt wie oben beschrieben transformiert sind, wobei das DNA-Konstrukt in einer transkriptionsfähigen Form vorliegt. Das DNA-Konstrukt kann beispielsweise episomal vorliegen oder stabil ins Chromosom integriert sein. Dabei können in der Zelle ein oder mehrere Kopien vorliegen. Falls eine RNA-Exportsequenz verwendet wird, die erst in Anwesenheit eines viralen Exportfaktors aktiv ist, so kann durch Transfektion oder Infektion der Zelle mit Expressionsbibliotheken nach viralen Faktoren gesucht werden, die mit der RNA-Exportsequenz interagieren oder weiter stromabwärts im RNA-Exportweg wirken, falls solche Faktoren noch nicht bekannt sind. Wird die RNA-Exportaktivität erfolgreich rekonstituiert, muss eine Steigerung der Expression des Rev-abhängigen Genes um mindestens das 2,5-fache, bevorzugt das 5-fache, nochmehr bevorzugt das 8-fache oder mehr zu messen sein.

Sind die Faktoren bereits bekannt, werden sie bevorzugt in der Zelle zur Verfügung gestellt, in der Regel in trans. Die Erfindung betrifft daher die Herstellung stabiler Zelllinien, die neben dem oben beschriebenen Exportkonstrukt auch noch das Gen für einen zusätzlichen viralen Exportfaktor chromosomal oder episomal integriert in sich tragen. Erfindungsgemäß kann jede eukaryontische Zelle verwendet werden, in der die für den RNA-Export erforderlichen Faktoren in Konzentrationen vorhanden sind, die dem natürlichen Infektionsmodell näherungsweise entsprechen. Außer für das weiter unten beschriebene Infektionsmodell ist es nicht erforderlich, dass die Zellen permissiv für eine replikative Infektion mit dem zu untersuchenden viralen System sind. Als eukaryontische Zellen können beispielsweise H1299-Zellen, HeLa-Zellen, HEK-Zellen (human embryonic kidney cells) verwendet werden.

Bei dem viralen Exportfaktor handelt es sich bevorzugt um Rev von HIV-1, HIV-2, SIV und EIAV oder Rex von HTLV-1 und HTLV-11, oder die 34K und E4orf6 Proteine von Adenoviren, oder EB2 Proteine von EBV, oder ORF 57 Genprodukte von Herpesvirus Saimiri, oder ICP 27 Proteine von HSV.

Noch ein weiterer Gegenstand der vorliegenden Erfindung ist die Herstellung stabiler Zelllinien, die neben dem Rev-abhängigen Gen zusätzlich ein oder mehrere konstitutiv exprimierende Gene beinhalten. Durch Bestimmen der Expressionsrate dieser Gene oder der Menge der zugehörigen Proteinprodukte kann man unterscheiden, ob ein zu testender Wirkstoffe spezifisch auf die vom in trans zur Verfügung gestellten Exportfaktor abhängige Expression einwirkt, oder nur allgemein die zelluläre Expression blockiert. Das vom viralen Exportfaktor in seiner Expression regulierte Reportergen und das konstitutiv exprimierte Protein müssen durch unterschiedliche Verfahren nachweisbar sein. Falls beide Proteine fluoreszierend sind, müssen sie sich also in der Wellenlänge des anregenden Lichts oder in der Wellenlänge des emittierten Lichts unterscheiden. Es ist aber auch möglich, andere enzymatische Verfahren oder die Erkennung eines anderen immunologischen Epitops zu verwenden. Das DNA-Reporterkonstrukt und eine mit der DNA transfizierbare Zelle oder eine mit dem DNA-Konstrukt bereits transfizierte Zelle können als Reagenzienkit zur Untersuchung von RNA-Exportvorgängen angeboten werden. Bevorzugt enthält der Reagenzienkit auch Leptomycin B, da mit dieser Substanz der RNA-Exportweg, der beispielsweise von HIV Rev abhängigen RNA-Konstrukten genutzt wird, spezifisch blockiert werden kann (Otero, 1998).

Noch ein weiterer Gegenstand der vorliegenden Erfindung ist ein Verfahren zum Nachweis einer erfolgten Virusinfektion, stärker bevorzugt einer retroviralen Virusinfektion, am meisten bevorzugt einer lentiviralen Virusinfektion. Der Nachweis beruht auf der Tatsache, dass in infizierten Zellen, nicht aber in nicht-infizierten Zellen ein für den Kernexport erforderlicher viraler Exportfaktor zur Verfügung steht. Falls es sich um eine Infektion mit HIV handelt, ist der virale RNA-Exportfaktor HIV Rev, bei einer Infektion mit HTLV ist der virale RNA-Exportfaktor HTLV Rex.

Wegen der leichten Verfügbarkeit von Patientenplasma bietet es sich an, den Infektionsnachweis durch den Nachweis infektiöser Viren im Plasma zu führen. Bei dieser Ausführungsform wird eine Zelle, die mit dem Reporterkonstrukt transfiziert ist, bevorzugt das Reporterkonstrukt stabil in sich trägt, besonders bevorzugt das Reporterkonstrukt stabil chromosomal integriert hat, mit dem Plasma des Patienten in Kontakt gebracht. Falls Viren im Plasma vorhanden sind, erfolgt eine Infektion der Zelle. Dadurch wird der notwendige, in trans aktive RNA-Exportfaktor zur Verfügung gestellt und der RNA-Export kann nachgewiesen werden.

Der Infektionsnachweis ist auch dann noch möglich, wenn keine Viren im Plasma mehr nachgewiesen werden können, aber dennoch Zellen mit dem Virus infiziert sind. In diesem Fall muss die Infektion dann von diesen Zellen ausgehen. Alternativ können die Zellen auch mit dem Reporterkonstrukt transfiziert werden.

Der Nachweis der Infektion kann, wenn das Reportergen geeignet gewählt wird, durch fluoreszente Bioanalytik erfolgen, gegebenenfalls durch die Autofluoreszenz des Reportergenproduktes, bevorzugt spektroskopisch, bevorzugt durch Fluoreszenzmikroskopie, noch mehr bevorzugt durch FACS Analysen. Dieser Nachweis kann manuell oder vollautomatisch in einer Hochdurchsatztestung erfolgen.

Der Nachweis einer Infektion durch Nachweis des vom viralen Exportfaktor, insbesondere Rev, abhängigen Reportergens kann zudem über die enzymatische Aktivität des gewähten Reportergenproduktes, wie der Phosphataseaktivität der SEAP erfolgen, der Acetyltransferaseaktivität des CAT Genproduktes, der Luciferaseaktivität des Luciferasegenes. Dieser Nachweis kann manuell oder vollautomatisch in einer Hochdurchsatztestung erfolgen.

Man kann aber auch die antigenen Eigenschaften eines Reporterproteins oder eines oder mehrerer Epitope des gewählten Reporterproteins ausnutzen. Der Nachweis erfolgt dann durch geeignete immunologische Methoden, wie ELISA, Immunfluoreszenz, Immunoblot, FACS immunologisch markierter Zellen. Er ist manuell oder vollautomatisch in einer Hochdurchsatztestung möglich.

Insbesondere konnte gezeigt werden, daß Zellen, die mit dem Rev-sensitiven hivGFP zusammen mit einem infektiösen proviralen HIV-1 Klon (HX10) transfiziert wurden, ebenfalls eine grün leuchtende Reportergen-Aktivität aufwiesen. Das hergestellte Rev-sensitive Reportersystem eignet sich also zur Detektion einer HIV-1 Genxpression und somit als lnfektionsnachweis.

Ein weiterer Nachweis des RNA-Exportnachweises nach Lu et al liegt in der Notwendigkeit, die Zellen zum Nachweis des Reporterproteins oder der Aktivität des Reporterproteins lysieren oder Fixieren zu müssen. Gegenstand der Erfindung ist daher weiterhin ein Verfahren zum Nachweis der RNA-Exports aus dem Zellkern, bei dem ein Reporterprotein verwendet wird, dass ohne Lyse oder Fixierung der Zellen nachgewiesen werden kann. Für diesen Zweck eignet sich beispielsweise ein fluoreszentes Protein. Ebenso kann aber auch ein geeigneter Selektionsmarker verwendet werden. Der Vorteil dieses Verfahrens gegenüber dem Stand der Technik liegt in der Möglichkeit, daß man
- (a): die Zellen mit einer Genbibliothek transfizieren oder infizieren kann
- (b): die Gene, die eine Modulation des RNA-Exports aus dem Zellkern bewirken, durch Kultivieren der Zellen und Isolation der Nukleinsäure aufreinigen kann.

### BEISPIELE:

### 1. Herstellung eines Rev-abhängigen GFP-Gens

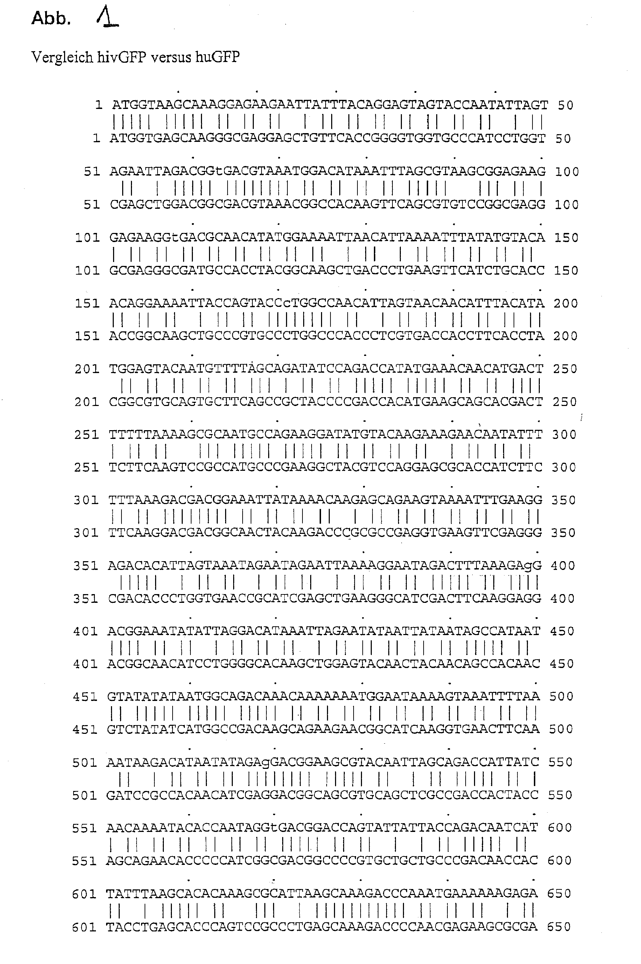

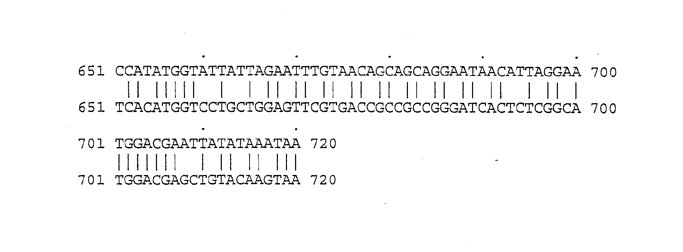

Der Leserahmen des green-fluorescent-protein (Gfp) Gens sollte unter Verwendung einer Kodonwahl wie sie in HIV-1 Strukturgenen zu finden ist künstlich aufgebaut werden. Dazu wurde die Aminosäuresequenz des Gfp Gens in eine entsprechende Nukleotidsequenz übersetzt. Dies wurde mit Hilfe des gcg Befehls "backtranslate" unter der Verwendung einer entsprechenden Matrix, wie sie in Tabelle 1 beschrieben ist, durchgeführt. Zur Subklonierung sowie zum Anfügen weiterer Sequenzelemente innerhalb nicht translatierter Regionen wurden weitere Schnittstellen eingefügt. Eine genaue Sequenz, inklusive der verwendeten Schnittstellen ist in SEQ ID Nr. 1 angegeben. Die so hergestellte Sequenz wurde als vollsynthetisches Gen unter Verwendung synthtetischer Oligonukleotide und einer bereits beschriebenen Methode {Zolotukhin, Potter, 1996} hergestellt. In Abbildung 1 ist ein Vergleich des hivGFP (Kodonwahl abgeleitet von HIV Strukturgenen) und huGFP (Kodonwahl abgeleitet von Säugergenen) abgebildet. Das so hergestellte GFP kodierende DNA Fragement ("hivGFP") wurde unter Verwendung der Schnittstellen Kpnl und Xhol in den Expressionsvektor pcDNA3.1 (+) (Stratagen, Heidelberg) unter die transkriptionelle Kontrolle des Cytomegalo-Virus (CMV) early promotor/enhancer gestellt ("pc-hivGFP"). Zur Herstellung eines analogen, jedoch in seiner kodonwahl dem humanen System angepaßten GFP Expressionsplasmides, wurde die kodierende Region des humanisierten GFP Genes (huGFP) mittels Polymerase Kettenreaktion (PCR) unter Verwendung der Oligonukleotide hu-1 und hu-2 aus einem komerzielle erhaltlichen Vektor amplifiziert und ebenfalls unter Verwendung der Schnittstellen Kpnl und Xhol in den Expressionsvektor pcDNA3.1(+) (Stratagen, Heidelberg) einkloniert ("pc-huGFP").

Wie in der Beschreibung dargelegt muß dem kodierenden Bereich ein isolierter (effizient nutzbarer) Spleißdonor (SD) vorangestellt werden, um eine Rev-Abhängigkeit des hivGFP Reporters zu erreichen. Der HIV-1 nichttranslatierte Bereich (UTR) innerhalb der späten HIV-1 Transkripte beinhaltet einen solchen SD. Diese Region wurde mittels PCR unter Verwendung der Oligonukleotide utr-1 und utr-2 aus proviraler HIV-1 DNA (HX10 siehe (Ratner et al., 1987)) amplifiziert und unter Verwendung der Schnittstellen Kpnl und Ncol direkt 5' vor das ATG der GFP-kodierenden Leserahmen der pc-huGFP und pc-hivGFP Konstrukte kloniert. Die resultierenden Konstrukte wurden im Weiteren als "pc-UTR-huGFP" bzw. "pc-UTR-hivGFP" bezeichnet.

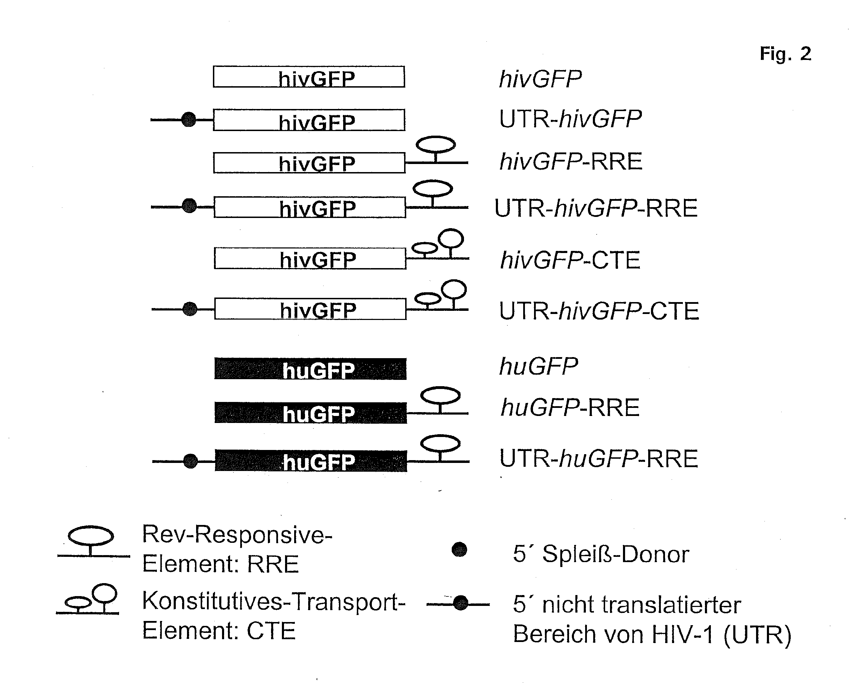

Wie in den Anwendungen dargestellt muß dem kodierenden Bereich eine RNA-Zielsequenz angefügt werden, um eine Rev-Abhängigkeit des hivGFP Reporters zu erreichen. Diese Zielsequenz interagiert auf RNA-Ebene entweder mit einem viralen Kernexportprotein (im Falle von HIV-1 das Rev-Protein) oder zellulären Kernexportproteinen. Aus diesem Grund wurde mittels PCR unter Verwendung der Oligonukleotide rre-1 und rre-2 das HIV-1 Rev-responsive-element aus proviraler HX10 DNA amplifiziert und unter Verwendung der Schnittstellen BamH1 und Xhol 3' hinter den GFP-kodierenden Bereich der pc-huGFP, pc-UTR-huGFP, pc-hivGFP, pc-UTR-hivGFP Konstrukte kloniert. Die resultierenden Konstrukte wurden im Weiteren als "pc-huGFP-RRE", "pc-UTR-huGFP-RRE", "pc-hivGFP-RRE", "pc-UTR-hivGFP-RRE" bezeichnet. Darüber hinaus wurde mittels PCR unter Verwendung der Oligonukleotide cte-1 und cte-2 das MPMV konstitutive Transportelement CTE aus proviraler MPMV DNA amplifiziert und unter Verwendung der Schnittstellen BamH1 und Xhol 3' hinter den GFP-kodierenden Bereich der pc-hivGFP, pc-UTR-hivGFP Konstrukte kloniert. Die resultierenden Konstrukte wurden im Weiteren als "pc-hivGFP-CTE", "pc-UTR-hivGFP-CTE" bezeichnet.In Abbildung 2 sind alle hergestellten GFP kodierenden Konstrukte schematisch dargestellt.

Für die Norhernblot Analysen wurde zudem die RRE kodierende Region in antisense Orientierung zum T7 Promotor in den pCR-Script Vektor (Stratagene, Heidelberg) einkloniert. Darüber hinaus wurde uns das pSP6-actin Konstrukt freundlicherweise von der Arbeitsgruppe F. Schwarzmann (IMMH, Regensburg) zur Verfügung gestellt. Zur Bereitstellung des viralen Rev Proteins in trans wurde uns freundlicherweise ein Rev-Expressionsplasmid von Prof. J. Hauber (Erlangen) zur verfügung gestellt.

### 2. Die Rev-abhängige GFP-Expression des hivGFP Reporters benötigt die Codonwahl von HIV Strukturgenen sowie den 5'-UTR/SD

Sämtliche Zellkulturprodukte waren von Life Technologies (Karlsruhe). Alle Säugerzellinien wurden bei 37øC und 5% CO₂ kultiviert. Die humane Lungenkarzinomzellinie H1299 wurden in Dulbecco's Modifiziertem Eagle Medium (DMEM) mit L-Glutamin, D-Glucose (4,5 mg/ml), Natriumpyruvat, 10% inaktiviertem fötalem Rinderserum, Penicillin (100 U/ml) und Streptomycin (100 µg/ml) gezogen. Die Zellen wurden nach Erreichen der Konfluenz im Verhältnis 1:10 subkultiviert.

1,5 * 10⁶ Zellen wurden in Petrischalen (Durchmesser: 100 mm) ausgesät und 24 h später durch Calciumphosphat Kopräzipitation (Graham and Eb, 1973) mit 30 µg Indikatorplasmid und 15 µg pc-Rev bzw. 15 µg pcDNA 3.1 Vektor transfiziert. Zellen und Kulturüberstände wurden 48 h nach der Transfektion geerntet. Die transfizierten Zellen wurden zweimal mit eiskaltem PBS (10 mM Na₂HPO₄ 1,8 mM KH₂PO₄ 137 mM NaCI, 2,7 mM KCI) gewaschen, in eiskaltem PBS abgekratzt, 10 Min. bei 300 g abzentrifugiert und in Lyse-Puffer (50 mM Tris-HCI, pH 8.0, 0.5% Triton X-100 (w/v)) 30 min auf Eis lysiert. Unlösliche Bestandteile des Zellysates wurden 30 min bei 10 000 g und 4°C abzentrifugiert. Die Gesamtproteinmenge des Überstandes wurde mit dem Bio-Rad Protein Assay (Bio-Rad, München) nach Herstellerangaben bestimmt.

Die Proben wurden mit gleichem Volumen an 2-fach Probenpuffer (Laemmli, 1970) versetzt und 5 min auf 95°C erhitzt. 50 µg Gesamtprotein aus Zellysaten oder der halbe Probenansatz aus angereicherten Überstanden (B.4.1) wurden über ein 12,5 %-iges SDS/Polyacrylamidgel aufgetrennt (Laemmli, 1970) auf eine Nitrocellulose-Membran elektrotransferiert und mit dem monoklonalen p²⁴-spezifischen Antikörper 13-5 (Wolf et al., 1990) analysiert und mittels einem sekundären, HRP (Meerrettich-Peroxidase: horse-radish-preoxidase) gekoppelten Antikörper detektiert und mittels chromogener Färbung nachgewiesen.

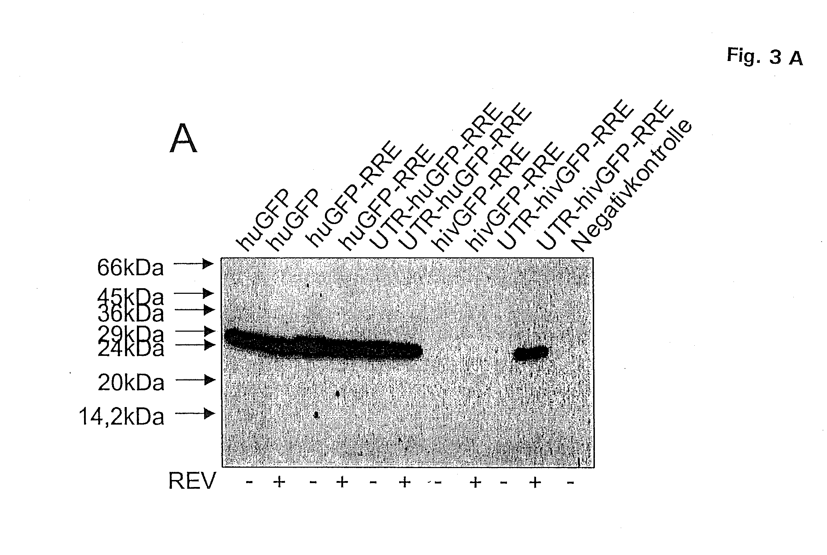

Die Reporterkonstrukte wurden transient in H1299 Zellen transfiziert. Die erzielte Expression wurde in An- und Abwesenheit von Rev/RRE als auch dem 5'-UTR/SD analysiert. Die huGFP-Expression konnte weder durch das Rev/RRE-System verstärkt werden (Abb. 3 A, Spuren 3, 4), noch wurde sie signifikant durch den 5'-UTR/SD (Abb. 3 A, Spur 5) beeinflußt. Auch die Kombination aus 5'-UTR/SD und Rev/RRE, konnte den synthetischen Leserahmen nicht in die Rev-Abhängigkeit überführen (Abb. 3 A, Spur 6). Die an die Kodonwahl von HIV-1 Strukturgenen angepaßten hivGFP Reporterkonstrukte verhielten sich jedoch im Gegensatz dazu völlig verschieden. So konnte in Abwesenheit des 5'-UTR/SD keine GFP Expression nachgewiesen werden, und zwar unabhängig von der An- oder Abwesenheit des Rev/RRE-Systems (Abb. 3 A, Spur 7,8). Nur in Anwesenheit des 5'-UTR/SD sowie des an HIV-1 Strukturgenen angepaßten hivGFP Reportergens konnte eine Rev/RRE abhängige Expression des GFP Reporters nachgewiesen werden (Abb. 3 A, vgl. Spur 9 mit 10).

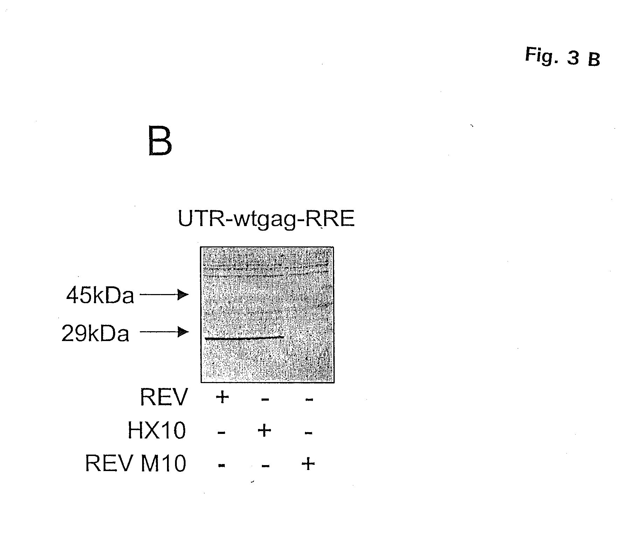

Eine Rev-abhängige Expression die auf den Kernexport viraler oder quasi-viraler Transkripte beruht sollte nicht durch eine einfache Stabilisierung der Transkripte durch die alleinige Rev/RRE Interaktion zurückzuführen sein. Die Rev-M10 Mutante kann zwar mit der RNA-Zielsequenz RRE und anderen Rev-Proteinen (mutierten wie Wildtyp) wechselwirken, jedoch durch einen Defekt innerhalb der Kernexportsignals (NES) nicht aus dem Kern exportiert werden (Stauber et al, 1995; Kubota et al, 1991). Es konnte keine GFP-Expression in Anwesenheit des Rev-M10 Proteins nach Transfektion mit dem UTR-hivGFP-RRE Reporter erzielt werden (Abb. 3 B, Spur 3). Die Rev-abhängige Expression des hivGFP Reporters ist also nicht auf eine Stabilisierung der RNA durch eine Rev/RRE Interaktion sondern auf den nachfolgenden Kernexport durch wildtyp Rev zurückzuführen. Zudem ließ sich durch den UTR-hivGFP-RRE Reporter eine GFP-Expression in Gegenwart kotransfizierter proviraler HIV-1 DNA (HX10) erzielen (Abb. 3 B, Spur 2). Es ist also möglich eine Hi-virale Infektion an Hand des während der viralen Replikation gebildeten Rev durch das hivGFP-Reportersystem nachzuweisen.

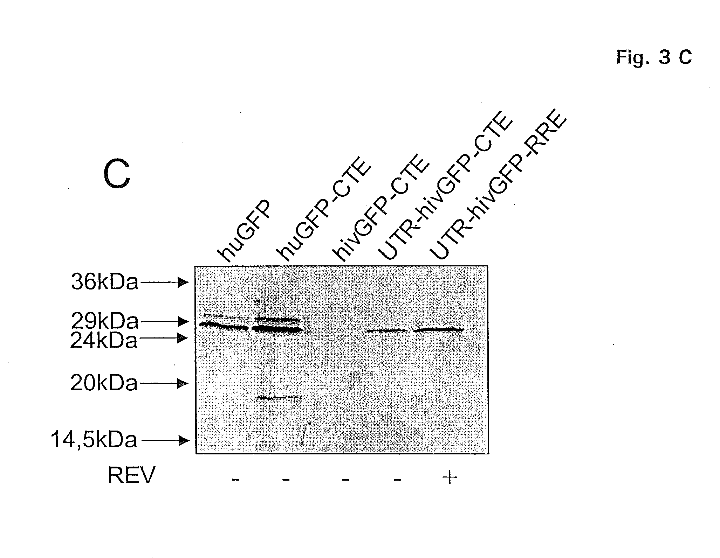

Darüber hinaus erwies sich die hivGFP-Expression unter Verwendung des heterologen CTE Kerntranslokationssystems ebenfalls als abhängig von der Präsenz des 5'-UTR/SD sowie der verwendeten Kodonwahl (Abb. 3 C). Dies zeigt, das dem auf dieser Erfindung basierenden Reporterssystem, ein übergeordnetes Kernrückhalteprinzip zur Grunde liegt welches unabhängig vom Kernexportmechanismus zum tragen kommt.

### 3. Die Rev-abhängige GFP-Expression des hivGFP Reporters basiert auf einer Rev-vermittelten Kerntranslokation

Die in Abbildung 3 zusammengefassten Experimente lassen auf einen Rev-vermittelten Kernexport der UTR-hivGFP-RRE Transkripte vermuten. Um diese Vermutung zu Belegen wurde die subzelluläre Verteilung der GFP-kodierenden Transkripte einer Northern Blot Analyse unterworfen. GFP-kodierende Transkripte wurde über eine RRE spezifische Sonde nachgewiesen. Als interne Kontrolle der Qualität und Quantität der RNA Präparation wurde zudem die Menge und Integrität der ß-Aktin RNA nachgewiesen.

Dazu wurden transfizierte Zellen abtrypsiniert und 2x mit eiskaltem PBS gewaschen. 1x10⁷ Zellen wurden mit 175µl Lyse-Puffer (50mM Tris, 140mM NaC1, 1.5mM MgCI₂, 0.5% NP-40, pH8.0) auf Eis 5 Min. partiell lysiert. Die cytoplasmatische Fraktion wurden von der Kernfraktion durch Zentrifugation abgetrennt (300 x g, 2 Min.) und auf Eis gestellt. Die Kerne wurden mit Lyse Puffer vorsichtig gewaschen und erneut zentrifugiert (300 x g, 2 Min.). Aus den Kernen und der cytoplasmatischen Fraktion wurde jeweils die Gesamt-RNA unter Verwendung des RNeasy-Kit (Qiagen, Hilden) präpariert. Die RNA-Präparationen wurden in RNAase-freiem Wasser aufgenommen und bei -80°C bis zur weiteren Verwendung aufbewahrt.

Folgende Lösungen wurden für die Northern Blot Analyse verwendet:

| | |
|---|---|
| SSPE (20x): | 175.3g NaC1, 27.6g NaH₂PO₄ H₂O 7.4g EDTA add 1l 1l H₂O (auf pH 7.4 einstellen) |
| MOPS (10x): | 83.72g MOPS, 8.23g NaAc, 20 ml EDTA (0.5 M) add 11 H2O (auf pH 7.0 einstellen) |
| SSC (20x): | 87.7g NaC1, 44.1 g NaCitrat add 500 ml H2O (auf pH 7.0 einstellen) |
| Denhards (100x): | 1g Ficoll (Typ400), 1g Polyvinylpyrrolidone, 1g BSA (Fraktion V) add 50 ml H2O |
| Hybridisierungspuffer: | 12.5 ml SSPE (20x), 25m1 Formamid, 2.5 Denhards (100x), 2.5 ml SDS (10%), 20mg tRNA |
| | (aus "brewers yeast", Boeringer Mannheim) |
| RNA Probenpuffer: | 10.0 ml Formamid, 3.5 ml Foraldehyd, 2.0 ml MOPS (5x) |
| RNA Auftragspuffer: | 50 % Gylcerol, 1 mM EDTA, 04 % Bromphenol-blau |

Die Northern Blot Analysen wurden angelehnt an das "Promega" Protokoll (RNA Applications Guide, Promega Corporation, Madison, USA) durchgeführt. 10 µl RNA-Präparation wurde mit 20 µl Probenpuffer und 5 µl Auftragspuffer versehen, und auf einem 1 %-igem Agarosegel (0.04M MOPS, 0.01 M NaAc, 0.001 M EDTA, 6.5% Formaldehyd, pH7.0) aufgetrennt. Das RNA-Gel wurde auf eine negativ geladene Nylonmembran (Boehringer, Mannheim) über Nacht mittels Kapillarkraft geblottet. Die RNA wurde auf der Membran durch UV Behandlung (1200 kJ für 1 Min.) fixiert und Nukleinsäuren wurden unspezifisch angefärbt (0,03% Methylenblau, 0,3M NaAc). Der Größenstandard wie auch die 18S und 28S RNA wurden angezeichnet und der Blot in Wasser entfärbt. Die Membran wurde 2 h bei 60°C in 10 ml Hybridisierungspuffer präinkubiert und über Nacht nach Zugabe einer radioaktiv markierten RNA-Sonde hybridisiert. Der Blot wurde dann nach mehrmaligem stringentem Waschen (0,1 SSC, 01% SDS) durch Exposition an eine Phosphor-Imager Platte und mit Hilfe der Molecular Analyst Software (Bio-Rad Laboratories, München) ausgewertet.

Radioaktive "antisense" RNA-Sonden zur spezifischen Detektion der RNA wurden durch in vitro Transkription unter Verwendung des Riboprobe in vitro Transcription Systems (Promega, Madison, USA) unter Beachtung der Herstellerangaben hergestellt. Als radioaktiv markiertes Nukleotid wurde ³²P-à-CTP (10 µCi pro Reaktion) verwendet. Als Transkriptionsmatrize wurden jeweils 500 ng linerisierte Plasmid DNA eingesetzt. Eine RRE-spezifische RNA-Sonde wurde durch T7 vermittelte Transkription von Xhol-linearisiertem pc-ERR hergestellt. Eine ß-Aktin spezifische RNA-Sonde wurde durch SP6 vermittelte Transkription von EcoRl-linearisiertem pSP6-actin hergestellt.

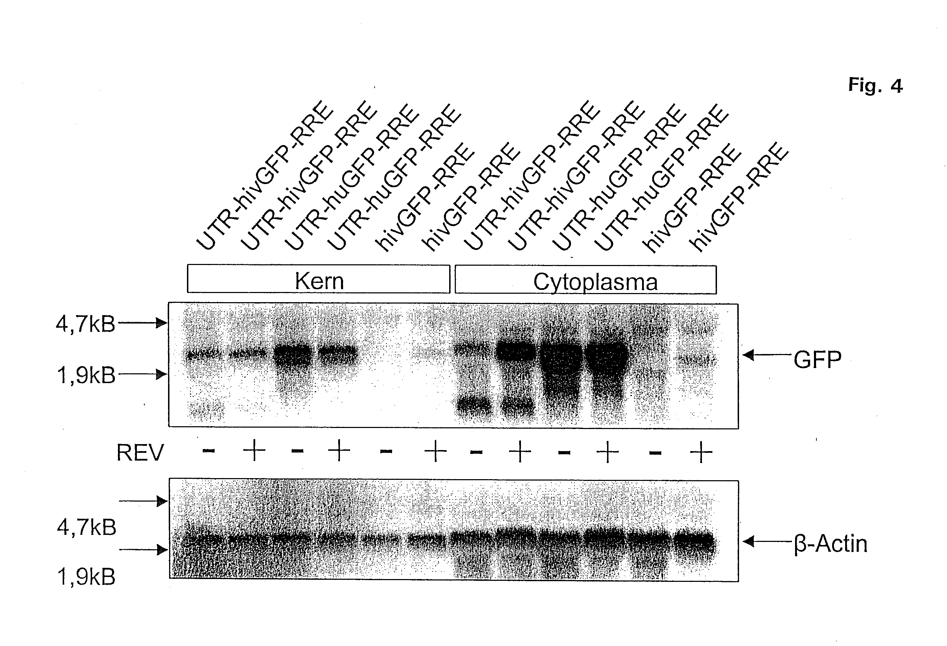

Die Mengen an cytoplasmatischer RNA korrelierten bei allen Reporterkonstrukten mit der gemessenen GFP-Expression (Abb. 4, Spuren 7 -12). In Abwesenheit des 5'-UTR konnten nur geringste Mengen an hivGFP-RNA (hivGFP-RRE) im Kern nachgewiesen werden, auch wenn Rev in trans zur Verfügung gestellt wurde (Abb. 4, Spur 5, 6). Im Gegensatz dazu akkumulierten in Anwesenheit des authentischen 5'-UTR (UTR-hivGFP-RRE) die an die Kodonwahl von HIV Strukturgenen angepaßten GFP-RNAs im Kern und waren dort in hohen Mengen nachweisbar (Abb. 4, Spur 1).

Ohne die Anpassung der Kodonwahl an HIV-1 Strukturgene erwies sich die huGFP-RNA als stabil im Kern und wurde konstitutiv in das Cytoplasma transportiert, auch wenn dem kodierenden Bereich der 5'-UTR/SD vorangestellt wurde. Durch das alleinige Voranstellen eines effizient genutzten SD in Abwesenheit eines (effizient genützten) SA, kann also ein beliebiges Gen nicht in die Rev-abhängigkeit überführt werden.

Die Rev-abhängige GFP-Expression von hivGFP kann mit Therapeutika, welche den Rev-vermittelten Kernexport blockieren gehemmt werden.

Wie in den Anwendungen beschrieben könnte das hier beschriebene Rev-abhängige hivGFP-Reporter System zur Identifizierung antiviral wirksamer Therapeutika, insbesondere solcher die den Kernexport der quasi-viralen GFP-kodierender RNA inhibieren, verwendet werden. Leptomycin B (LMB) und die transdominant-negative Rev-Mutante M10 (Rev M10) sind die bekanntesten Hemmstoffe eines Rev-vermittelten Kernexportes. Diese etablierten Wirkstoffe sollten ebenfalls inhibierend auf die Rev-abhängige Expression des hivGFP-Reporters einwirken. Um dies zu testen, wurde die hivGFP-Expression in Anwesenheit von Rev und LMB bzw. Rev M 10 analysiert.

Für die Rev M10 Experimente wurden 3 x 10⁵ H1299 Zellen in einer 6-Napf Zellkulturplatte ausgesät und 24 h später durch Calciumphosphat Kopräzipitation mit 5 µg Reporterplasmid, 2,5 µg pc-Rev und ansteigenden Mengen an pc-RevM10 transfiziert. Zudem wurde der Transfektionsansatz mit pcDNA 3.1 Plasmid DNA auf insgesamt jeweils 15 µg Gesamt DNA eingestellt. Für die LMB Experimente wurden 3 x 10⁵ H1299 Zellen in einer 6-Napf Zellkulturplatte ausgesät und 24 h später durch Calciumphosphat Kopräzipitation mit 10 µg Reporterplasmid sowie 5 µg pc-Rev oder pcDNA 3.1 Plasmid DNA transfiziert. 24 h vor der Ernte wurde das Medium mit 5 nM an LMB supplementiert. Die Zellen wurden geerntet und die GFP-Expression ausgelesen wie oben beschrieben.

Schon durch die Ko-Transfektion von "quimotaren Mengen an RevM10 konnte die Rev-abhängige Expression des UTR-hivGFP-RRE Reporterkonstruktes stark reduziert werden (Abb. 5 A). Ebenso konnte nur die Expression des Rev-abhängigen GFP-Reporters durch die Anwesenheit von 5 nM LMB inhibiert werden, jedoch nicht die Expression des huGFP Reporters (Abb. 5 B). Damit konnte belegt werden, das die hivGFP RNA den Zellkern auf dem gleichen Kernexportweg (CRM1) verläßt wie späte HIV-1 Transkripte. Darüber hinaus konnte belegt werden, daß sich GFP-Expression des etablierten Rev-abhängigen GFP-Reporters durch Rev-Inhibitoren in gleicher Weise hemmen läßt, wie die Expression später HIV-1 Gene. Durch den einfachen autofluoreszenten Nachweis des GFP-Reporters eignet sich dieses somit zur Identifikation antiviral wirksamer Rev-Inhibitoren. Die Rev-abhängige GFP-Expression des hivGFP Reporters kann auf Einzelzellbasis detektiert werden und ist mittels Durchfluß-Zytometrie quantifizierbar.

Die autofluoreszente Eigenschaften des GFP erlauben die Detektion der Rev-abhängigen Expression des hivGFP-Reportersystems auf Einzelzellbasis. Zur mikroskopischen Deketion wurden dazu sterile Obejektträger in Petrischalen gelegt, 10⁶ H1299 Zellen darauf ausgesät und und 24 h später durch Calciumphosphat Kopräzipitation mit 30 µg GFP-Reporter Plasmid und 15 µg pc-Rev oder 15 µg pcDNA 3.1 (+) transfiziert. Nach 48 h wurden die Objektträger 2x mit PBS gewaschen, mit 4% Paraformaldehyd fixiert (10 Min.) und anschließend für 1 h mit DAPI (1mg/ml) bei 37°C gefärbt. Die mikroskopische Detektion des GFP-Genproduktes erfolgte mit Hilfe eines Olympus AX500-Fluoreszenzmikroskopes.

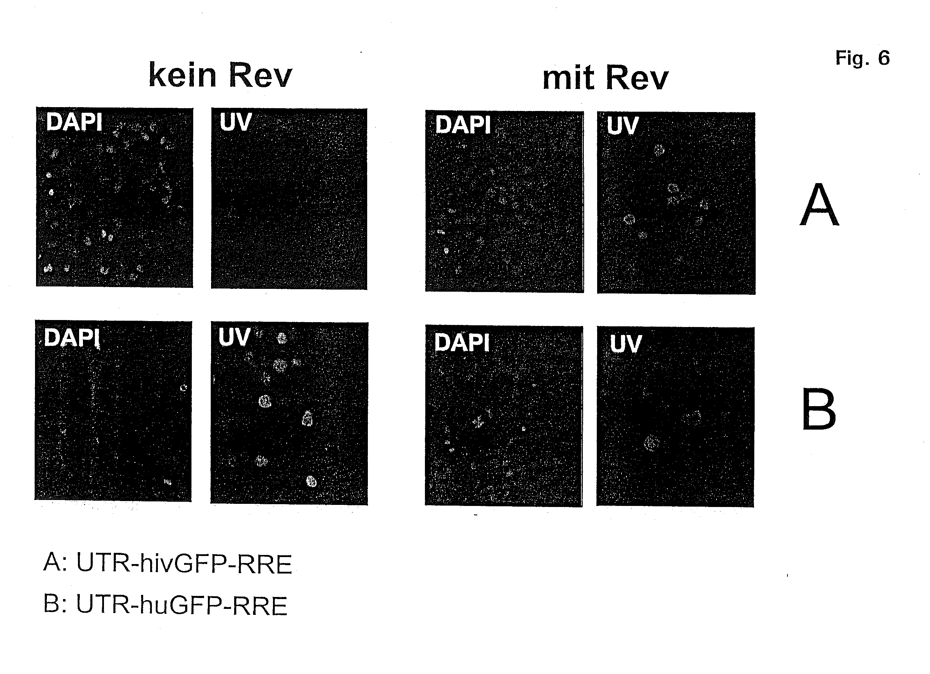

Bei dem auf huGFP basierenden Reporter war unbeiflußt von der An- und Abwesenheit von Rev eine GFP Aktivität nachweisbar. Im Gegensatz war nur dann in UTR-hivGFP-RRE transfizierten Zellen die grün leuchtende Reporteraktivität des GFP nachweisbar wenn Rev kotransfiziert wurde (Abb. 6). Ein Nachweis des Rev-abhängigen GFP-Reporters kann also auf Einzelzellbasis erfolgen.

Um dies besser quantifizieren zu können, wurden zudem die GFP-Aktivität transfizierter Zellen einer FACS Analyse unterworfen. Für die Analyse im Durchflußzytometer wurden 10⁶ H1299 Zellen in Petrischalen ausgesät und 24 h später durch Calciumphosphat Kopräzipitation mit 30 µg Reporterkonstrukt und 15 µg pc-Rev bzw. pcDNA 3.1 (+) transfiziert. Die transfizierten Zellen wurden 48 h später abtrypsiniert, in eiskalten PBS aufgenommen und einer FACS-Analyse unterworfen. Die Ergebnisse wurden in einem 2-dimensionalen Diagramm dargestellt. Dazu wurde die Fluoreszenzintensität (x-Achse) gegen die Zellzahl (y-Achse) aufgetragen.

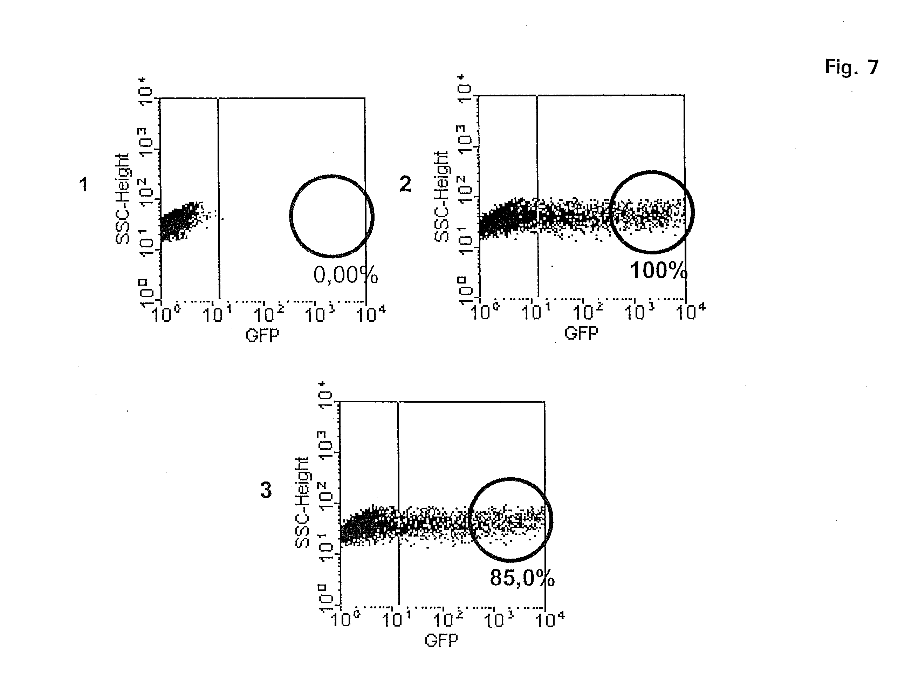

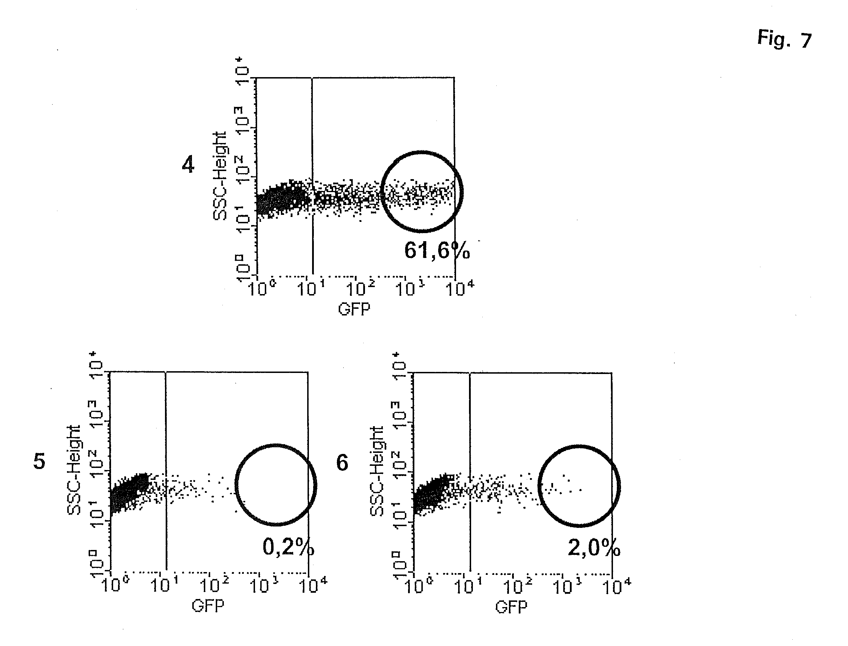

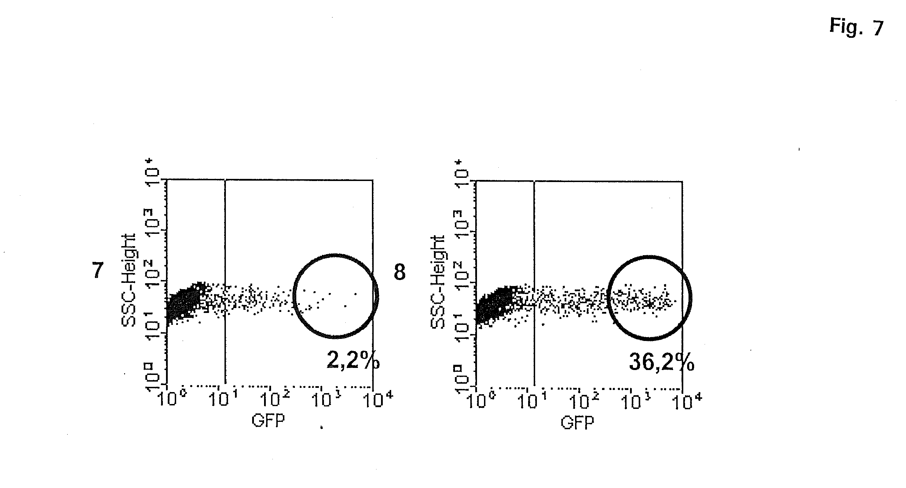

Die Ergebnisse aus den Expressionsanalysen unter Verwendung der Western Blot Technik konnten durch die FACS analysen bestätigt werden. Wiederum bewirkte das Rev/RRE System keine Steigerung der GFP-Expression, unabhängig von der An- oder Abwesenheit des 5'-UTR (Abb. 7, 2 bis 4). Nur in den GFP-Reporterkonstrukten deren Kodonwahl an die von HIV-1 Strukturgenen angepaßt wurde und gleichzeitig einen 5'-UTR/SD aufweisen konnte eine Rev/RRE-abhängige GFP-Reporter Aktivität nachgewiesen werden. In Anwesenheit von Rev und RRE konnte die GFP-Aktivität des hivGFP-Reporters von einer kaum Detektierbaren Hintergrundaktivität (2,2% von huGFP) auf den mehr als 15-fachen Wert (36,2% von huGFP) gesteigert werden. Das basierend auf dieser Erfindung hergestellte GFP-Reporterkonstrukt erlaubt demnach die quantitative Detektion eines Rev-vermittelten Kernexportes auf Einzelbasis und eignet sich somit zur Hochdurchsatztestung.

Sequenzen:
utr-1: gat cga att ccg acg cag gac tcg gct tgc
utr-2: gat ccc atg gct ctc tcc ttc tag cct ccg
rre-1: gat cgg atc cga gat ctt cag acc tgg agg ag
rre-2: gat cct cga ggt tca cta atc gaa tgg atc tg
hu-1: gat cga att caa cca tgg tga gca agg gcg agg ag
hu-2: gat cct cga gaa gga tcc ttt act tgt aca gct cgt c
cte-1: gct agg atc ccc att atc atc gcc tgg aac
cte-2: cga act cga gca aac aga ggc caa gac atc
**Tabelle 1:**

**Tabelle 1: Bevorzugte Codonwahl zur Herstellung eines rev-abhängigen Gens**

| Aminosäure | Kodon | Priorität | Aminosäure | Kodon | Priorität |
|---|---|---|---|---|---|
| Ala | GCA | 1 | Ile | ATA | 1 |
| Ala | GCT | 2 | Ile | ATT | 2 |
| Arg | AGG | 2 | Leu | TTA | 1 |
| Arg | AGA | 1 | Leu | CTA | 2 |
| Asn | AAT | 1 | Lys | AAG | 2 |
| Asn | AAC | 2 | Lys | AAA | 1 |
| Asp | GAT | 2 | Met | ATG | 1 |
| Asp | GAC | 1 | Phe | TTT | 1 |
| Cys | TGT | 1 | Phe | TTC | 2 |
| Cys | TGC | 2 | Pro | CCA | 1 |
| End | TAG | 2 | Pro | CCT | 2 |
| End | TAA | 1 | Ser | AGC | 1 |
| Gln | CAG | 2 | Ser | TCA | 2 |
| Gln | CAA | 1 | Thr | ACA | 1 |
| Glu | GAG | 2 | Thr | ACT | 2 |
| Glu | GAA | 1 | Trp | TGG | 1 |
| Gly | GGG | 2 | Tyr | TAT | 1 |
| Gly | GGA | 1 | Tyr | TAC | 2 |
| His | CAT | 1 | Val | GTA | 1 |
| His | CAC | 2 | Val | GTG | 2 |

| | | | | | |
|---|---|---|---|---|---|
| Priorität 1: bevorzugtes Codon Priorität 2: bevorzugtes Codon, falls Codon der Priorität 1 nicht verwendet wird. | | | | | |

### Literatur:

Ausubel, F.M., Brent, R., Kingston, R.E., Moore, D.D., Seidman, J.G., Smith, J.A., and Struhl, K.(1994). Percentage of Codon Synonomous Usage and Frequency of Codon Occurrence in Various Organisms. Current Protocols in Molecular Biology 2, A1.8-A1.9
Borg, K.T., Favaro, J.P., and Arrigo, S.J.(1997). Involvement of human immunodeficiency virus type-1 splice sites in the cytoplasmic accumulation of viral RNA. Virology 236, 95-103.
Chang, D.D. and Sharp, P.A.(1989). Regulation by HIV Rev depends upon recognition of splice sites. Cell 59, 789-795.
Chen, C.Y. and Shyu, A.B.(1995). AU-rich elements: characterization and importance in mRNA degradation. Trends.Biochem.Sci. 20, 465-470.
Cochrane, A.W., Jones, K.S., Beidas, S., Dillon, P.J., Skalka, A.M., and Rosen, C.A.(1991). Identification and characterization of intragenic sequences which repress human immunodeficiency virus structural gene expression. J.Virol. 65, 5305-5313.
Graham, F.L. and Eb, A.J.(1973). A new technique for the assay of infectivity of human adenovirus 5 DNA. Virology 52, 456-467.
Kaufmann, R. (1979). High level production of proteins in mammalian cells. Genetic engineering, eds. Setlow, J. K. and Hollaender, A., New York, Plenum Press, 155-198.
Kjems, J., Frankel, A.D., and Sharp, P.A.(1991). Specific regulation of mRNA splicing in vitro by a peptide from HIV-1 Rev. Cell 67, 169-178.
Kjems, J. and Sharp, P.A.(1993). The basic domain of Rev from human immunodeficiency virus type 1 specifically blocks the entry of U4/U6.U5 small nuclear ribonucleoprotein in spliceosome assembly. J.Virol. 67, 4769-4776.
Kubota, S., Nosaka, T., Furuta, R., Maki, M., Hatanaka, M. (1991). Functional conversion from HIV-1 Rev to HTLV-1 Rex by mutation. Biochem. Biophys. Research Commun. 178:1226-1232
Laemmli, U.K.(1970). Cleavage of structural proteins during the assembly of the head of bacteriophage T4. Nature 227, 680-685.
Lu, X.B., Heimer, J., Rekosh, D., and Hammarskjold, M.L.(1990). U1 small nuclear RNA plays a direct role in the formation of a rev- regulated human immunodeficiency virus env mRNA that remains unspliced. Proc.Natl.Acad.Sci.U.S.A. 87, 7598-7602.
Luo, Y., Yu, H., and Peterlin, B.M.(1994). Cellular protein modulates effects of human immunodeficiency virus type 1 Rev. J.Virol. 68, 3850-3856.
Maldareili, F., Martin, M.A., and Strebei, K.(1991). Identification of posttranscriptionally active inhibitory sequences in human immunodeficiency virus type 1 RNA: novel level of gene regulation. J.Virol. 65, 5732-5743.
Malim, M.H. and Cullen, B.R.(1993). Rev and the fate of pre-mRNA in the nucleus: implications for the regulation of RNA processing in eukaryotes. Mol.Cell Biol. 13, 6180-6189.
Mikaelian, 1., Krieg, M., Gait, M.J., and Karn, J.(1996). Interactions of INS (CRS) elements and the splicing machinery regulate the production of Rev-responsive mRNAs. J.Moi.Bioi. 257, 246-264.
Nasioulas, G., Zolotukhin, A.S., Tabernero, C., Solomin, L., Cunningham, C.P., Pavlakis, G.N., and Felber, B.K.(1994). Elements distinct from human immunodeficiency virus type 1 splice sites are responsible for the Rev dependence of env mRNA. J.Virol. 68, 2986-2993.
O'Reilly, M.M., McNally, M.T., and Beemon, K.L.(1995). Two strong 5' splice sites and competing, suboptimal 3' splice sites involved in alternative splicing of human immunodeficiency virus type 1 RNA. Virology 213, 373-385.
Olsen, H.S., Cochrane, A.W., and Rosen, C.(1992). Interaction of cellular factors with intragenic cis-acting repressive sequences within the HIV genome. Virology 191, 709-715.
Otero, G. C., Harris, M. E., Donello, J. E. and Hope, T. J. (1998). Leptomycin B inhibits equine infectious anemia virus Rev and feline immunodeficiency virus Rev function, but not the function of the hepatitis B virus posttranscriptional regulatory element. J. Virol. 72, 7593-7597.
Pollard, V.W. and Malim, M.H.(1998). The HIV-1 Rev protein [In Process Citation]. Annu.Rev.Microbiol. 52:491-532, 491-532.
Powell, D.M., Amaral, M.C., Wu, J.Y., Maniatis, T., and Greene, W.C.(1997). HIV Rev-dependent binding of SF2/ASF to the Rev response element: possible role in Rev-mediated inhibition of HIV RNA splicing. Proc.Natl.Acad.Sci.U.S.A. 94, 973-978.
Ratner, L., Fisher, A., Jagodzinski, L.L., Mitsuya, H., Liou, R.S., Gallo, R.C., and Wong Staal, F.(1987). Complete nucleotide sequences of functional clones of the AIDS virus. AIDS Res.Hum.Retroviruses 3, 57-69.
Rosen, C.A., Terwilliger, E., Dayton, A., Sodroski, J.G., and Haseltine, W.A. (1988). Intragenic cis-acting art gene-responsive sequences of the human immunodeficiency virus. Proc.Natl.Acad.Sci.U.S.A. 85, 2071-2075.
Schneider, R., Campbell, M., Nasioulas, G., Felber, B.K., and Pavlakis, G.N. (1997). Inactivation of the human immunodeficiency virus type 1 inhibitory elements allows Rev-independent expression of Gag and Gag/protease and particle formation. J.Virol. 71, 4892-4903.
Schwartz, S., Campbell, M., Nasioulas, G., Harrison, J., Felber, B.K., and Pavlakis, G.N.(1992a). Mutational inactivation of an inhibitory sequence in human immunodeficiency virus type 1 results in Rev-independent gag expression. J.Virol. 66, 7176-7182.
Schwartz, S., Felber, B.K., and Pavlakis, G.N.(1992b). Distinct RNA sequences in the gag region of human immunodeficiency virus type 1 decrease RNA stability and inhibit expression in the absence of Rev protein. J.Virol. 66, 150-159.
Stauber, R., Gaitanaris, G. A., and Pavlakis, G. N. (1995). Analysis of trafficing of Rev and transdominant Rev-proteins in living cells using green fluorescent protein fusions: transdominant Rev blocks the export of Rev from the nucleus to the cytoplasm. Virology 213, 439-449.

### Abbildungen

Abb. 1: Dargestellt ist ein Sequenzvergleich des humanisierten GFP Gens versus dem hivGFP Gens dessen Kodonwahl an die von HIV-1 Strukturgene angepaßt wurde. Fast jede dritte wobbie Position wurde zumeist durch ein A oder T ersetzt. Damit sinkt die Homologie der beiden Leserahmen auf 67.9%. Der Gesamt AT-Gehalt stieg damit von ca. 37% (huGFP) auf ca. 69% (hivGFP) an, ohne jedoch die Aminosäure Sequenz der resultiernden Genprodukte zu ändern.
Abb. 2: Schematische Darstellung aller in dieser Arbeit hergestellten und verwendeten GFP-Reporterkonstrukte. Offene Kästen symbolisieren an HIV Strukturgene angepaßte GFP-Gene (hivGFP), schwarz hinterlegte Kästen symbolisieren an Säugergene angepaßte GFP-Gene (huGFP). Waagrechte Striche symbolisieren nichttranslatierte Regionen. Alle weiteren Symbole sind unten im Bild erklärt.
Abb. 3: Expressionanalyse der synthetischen Leserahmen. H1299 Zellen wurden mit den angegebenen GFP-Konstrukten transfiziert, sowie mit den unterhalb der Bilder angebenen Vektoren (+) oder leerem Vektor (-) kotransfiziert. Die GFP-Produktion wurde durch konventioneller Immunoblot Analyse nachgewiesen. (A) Austestung einer Rev-abhängigen GFP Expression. (B) Austestung der GFP Expression des UTR-hivGFP-RRE in Anwesenheit von Rev, einer mutierten Form von Rev (Rev M10) und proviraler HIV-1 DNA (HX10). (C) Austestung einer CTE-vermittelten GFP Expression der Reporterkonstrukte. Position und Molekulargewicht des GF-Proteins sind am rechten Rand mittels Pfeil angegeben.
Abb. 4: Northern Blot Analyse und subzelluläre Verteilung GFP-kodierender RNAs. Transfizierte H1299 Zellen wurden partiell mit 0,5% NP-40 Puffer lysiert und die Kerne vom Cytoplasma durch Zentrifugation getrennt. Aus der nukleären und cytoplasmatischen Fraktion wurde jeweils die Gesamt-RNA präpariert und einer Northern Blot Anaylse unterworfen. An HIV-1 Strukturgene adaptierte und an Säugergene angepaßte GFP-Transkripte konnten mittels einer RRE spezifischen Sonde gleichzeitig detektiert werden. Größe und Position der GFP-RNA ist mit Pfeil angegeben. Als interne Kontrolle wurde ebenfalls die RNA des Haushaltsgen ß-Aktin nachgewiesen.
Abb.5: Einfluß von Rev-Inhibitoren auf die Expression der GFP-Reporter. H1299 Zellen wurden mit den angegebenen GFP-Konstrukten transfiziert und im Western Blot Analysiert. (A) Zur Austestung des Einflusses der transdominanten Rev-Mutante (Rev M10) auf die GFP-Expression des UTR-hivGFP-RRE Reporters wurden 5 µg des Reporters mit jeweils mit 2,5 µg Rev-Plasmid sowie ansteigenden Mengen an Rev M10 Expressionsplasmid (0; 2,5; 5; 7,5) kotransfiziert. (B) Zur Austestung des Einflusses von LMB auf die GFP Expression wurden die angegebenen Konstrukte in An- (+) und Abwesenheit (-) von 5 nM LMB, sowie Rev (+) und leerem Vektor (-) kotransfiziert.
Abb. 6: Immunfluoreszenz Analyse des huGFP und hivGFP Reporters. H1299 Zellen wurden auf Objekträgern mit den angegebenen Konstrukten transfiziert und mit Rev oder leerem Vektor (kein Rev) kotransfiziert. Nach 48 h wurden die Zellen fixiert und mit DAPI gefärbt und die autofluoreszente Aktivität des GFP Genproduktes an einem Immunfluoreszenz Mikroskop ausgewertet.
Abb. 7: Durchfluß-Zytometrie Analyse transfizierter H1299 Zellen. 1: Mock, 2: huGFP, 3: UTR-huGFP-RRE, 4: UTR-huGFP-RRE und REV., 5: hivGFP-RRE, 6: hivGFP-RRE und REV, 7: UTR-hivGFP-RRE, 8: UTR-hivGFP-RRE und REV. Die Y-Achse gibt die Streulicht Intensität an, die X-Achse gibt die GFP-bedingte Fluoreszenz an. Die vertikale Linie teilt die Zellpopulationen in (links) nicht-fluoreszent und meßbare fluoreszent GFP-Aktivität (rechts) ein. Die prozentualen Mengen an Zellen sind oben in den Analysen angegeben. Die roten Kreise beinhalten Zellpopulation mit hoher fluoreszenter GFP-Aktivität. Die prozentualen Mengen an diesen Zellen sind unten, in den Analysen angegeben (rot markiert).

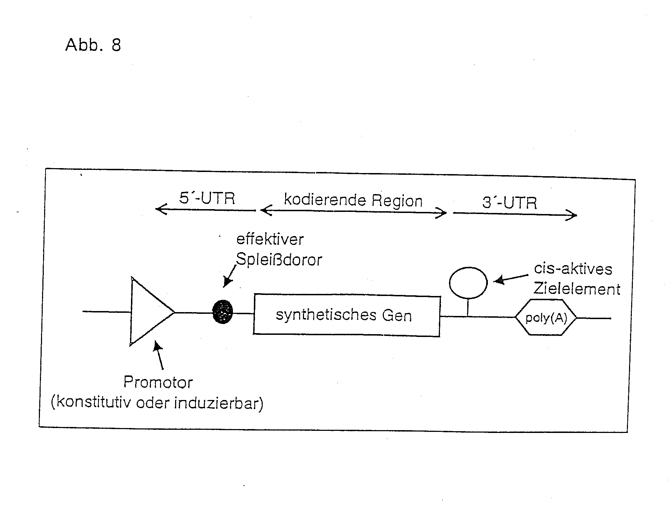

Abb. 8: Schematische Darstellung eines Rev-abhängigen Genes. Das synthetische Gen muß eine für Säugergene ungewöhnliche Codonwahl aufweisen oder einen durchweg hohen 50%) A/T Gehalt. 5' von diesem Gen, ist eine nicht translatierte Region (UTR) positioniert, die einen effektiven Spleißdonor beinhaltet. 3' von diesem Gen muß die virale Zielsequenz positioniert sein. Bei dieser Zielsequenz kann es entweder um ein konstitutives Transportelement wie des MPMV CTE handeln, oder um die Zielsequenz eines viralen RNA Transportmoleküls, wie des HIV-1 RRE. Zelluläre oder virale Exportfaktoren vermitteln dann den Kernexport. Das Rev-abhängige Gen steht unter der transkriptionellen Kontrolle eines induzierbaren (Tet On/Off) oder konstitutiven (CMV, SV40) Promotors. Die Polyadenylierung der Transkripte wird durch ein Polyadenylierungssignal wie des BGHpoly(A) Signals gewährleistet.

### Die folgenden Punkte sind Teil der Anmeldung

1. Verfahren zum Nachweisen von RNA-Export aus dem Zellkern einer eukaryontischen Zielzelle, umfassend die Schritte:
   (a) Bereitstellen einer Nukleinsäure, die für ein Reporterprotein kodiert und deren Transkript abhängig vom Vorhandensein
      (i)eines cis-aktiven RNA-Exportsignals in operativer Verknüpfung mit der Nukleinsäure und
      (ii) eines trans-aktiven Faktors und gegebenenfalls
      (iii) eines funktionalen 5'-Spleißdonors in Abwesenheit eines funktionalen 3'-Spleißakzeptors
      aus dem Zellkern exportiert wird,
   (b) Einbringen der Nukleinsäure in den Zellkern der Zielzelle, so dass sie dort in operativer Verknüpfung mit einer Transkriptionskontrollsequenz vorliegt, und dass bei Transkription der Nukleinsäure ein Transkript erzeugt wird, wobei der für das Reporterprotein kodierende Abschnitt des Transkripts keinem Spleißvorgang unterzogen werden kann,
   (c) Transkribieren der Nukleinsäure und
   (d) Bestimmen, ob das resultierende Transkript aus dem Zellkern exportiert wird.
2. Verfahren nach Punkt 1 zur Identifizierung von cis-aktiven RNA-Exportelementen.
3. Verfahren nach Punkt 1 zur Identifizierung von trans-aktiven RNA-Exportfaktoren.
4. Verfahren nach Punkt 1 zur Identifizierung von Molekülen, die den RNA-Export beeinflussen.
5. Verfahren nach Punkt 4,
   dadurch gekennzeichnet, dass
   es sich bei dem zu identifizierenden Molekül um ein kleines Molekül mit einer Molekularmasse kleiner 2000 Dalton, um DNA, RNA, Derivate oder Mimetika davon, um ein Peptid, ein modifiziertes Peptid, ein Protein oder ein modifiziertes Protein handelt.
6. Verfahren nach einem der vorhergehenden Punkte,
   dadurch gekennzeichnet, dass
   die RNA-Exportabhängigkeit der Nukleinsäure durch eine Codonwahl erreicht wird, die die metabolische Stabilität der Reporter-RNA herabsetzt.
7. Verfahren nach einem der vorhergehenden Punkte,
   dadurch gekennzeichnet, dass
   die RNA-Exportabhängigkeit der Nukleinsäure durch eine zumindest teilweise Anpassung der Codonwahl der Nukleinsäure an die Codonwahl exportierter viraler RNA erreicht wird.
8. Verfahren nach einem der vorhergehenden Punkte,
   dadurch gekennzeichnet, dass
   die RNA-Exportabhängigkeit der Nukleinsäure durch eine zumindest teilweise Anpassung der Codonwahl der Nukleinsäure an die Codonwahl von HIV-1 (human immunodeficiency virus 1) erreicht wird.
9. Verfahren nach einem der vorhergehenden Punkte,
   dadurch gekennzeichnet, dass
   die RNA-Exportabhängigkeit der Nukleinsäure durch eine Erhöhung des A/T-Anteiis der Nukleinsäure erreicht wird.
10. Verfahren nach einem der vorhergehenden Punkte,
   dadurch gekennzeichnet, dass
   es sich bei der Nukleinsäure um eine synthetisch hergestellte Nukleinsäure handelt.
11. Verfahren nach einem der vorhergehenden Punkte,
   dadurch gekennzeichnet, dass
   es sich bei dem Reportergen um ein Gen für ein fluoreszierendes Protein handelt.
12. Verfahren nach Punkt 11,
   dadurch gekennzeichnet, dass
   es sich bei dem fluoreszierenden Protein um GFP (green fluorescent protein), BFP (blue fluorescent protein), RFP (red fluorescent protein), YFP (yellow fluorescent protein), eGFP (enhanced green fluorescent protein), eBFP (enhanced blue fluorescent protein), eRFP (enhanced red fluorescent protein), eYFP (enhanced yellow fluorescent protein) oder hrGFP handelt.
13. Verfahren nach einem der vorhergehenden Punkte,
   dadurch gekennzeichnet, dass
   das Genprodukt des Reportergens ein Protein ist, dessen enzymatische Aktivität nachweisbar ist.
14. Verfahren nach Punkt 13,
   dadurch gekennzeichnet, dass
   es sich bei dem Genprodukt um LUC (Luziferase), AP (alkalische Phosphatase), SEAP (sekretorische alkalische Phosphatase) oder CAT (Chloramphenicol Acetyltransferase) handelt.
15. Verfahren nach einem der vorhergehenden Punkte,
   dadurch gekennzeichnet, dass
   es sich bei dem Reporterprotein um ein immunologisch nachweisbares Protein handelt.
16. Verfahren nach einem der vorhergehenden Punkte,
   dadurch gekennzeichnet, dass
   es sich bei dem Reporterprotein um einen Selektionsmarker handelt.
17. Verfahren nach einem der vorhergehenden Punkte,
   dadurch gekennzeichnet, dass
   es sich bei dem Reporterprotein um ein Regulatorgen, insbesondere einen Transkriptionsfaktor handelt.
18. Verfahren nach einem der vorhergehenden Punkte,
   dadurch gekennzeichnet, dass
   die Nukleinsäure ein cis-aktives RNA-Exportelement enthält.
19. Verfahren nach Punkt 18,
   dadurch gekennzeichnet, dass
   es sich bei dem cis-aktiven RNA-Exportelement um ein konstitutives RNA-Exportelement handelt.
20. Verfahren nach Punkt 19,
   dadurch gekennzeichnet, dass
   es sich bei dem cis-aktiven RNA-Exportelement um MPMV CTE (Mason Pfizer monkey virus constitutive transport element), RSV CTE (Rous sarkoma virus constitutive transport element) oder SRV CTE (simian retrovirus constitutive transport element) handelt.
21. Verfahren nach Punkt 18,
   dadurch gekennzeichnet, dass
   es sich bei dem cis-aktiven RNA-Exportelement um ein Exportelement handelt, das durch einen viralen Exportfaktor erkannt wird.
22. Verfahren nach Punkt 21,
   dadurch gekennzeichnet, dass
   es sich bei dem cis-aktiven RNA-Exportelement um HIV-1 RRE (human immunodeficiency virus 1 Rev responsive element), HIV-2 RRE (human immunodeficiency virus 2 Rev responsive element), SIV RRE (simian immunodeficiency virus Rev responsive element), HTLV-1 (human T-cell leukemia virus 1) Rex responsive element oder HTLV-11 (human T-cell leukemia virus 11) Rex responsive element handelt.
23. DNA-Sequenz, die für ein Reporterprotein kodiert und in operativer Verknüpfung mit einem cis-aktiven RNA-Exportelement und gegebenenfalls mit einem funktionalen 5'-Spleißdonor in Abwesenheit eines funktionalen 3'-Spleißakzeptors vorliegt,
   dadurch gekennzeichnet, dass
   (a) die für das Reporterprotein kodierende DNA-Sequenz gegenüber der Wildtypsequenz auf Nukleinsäureebene modifiziert ist,
   (b) für die modifizierte DNA-Sequenz ein RNA-Export in Abhängigkeit vom cis-aktiven RNA-Exportelement erfolgt und
   (c) für die Wildtyp DNA-Sequenz im wesentlichen kein RNA-Export in Abhängigkeit des cis-aktiven RNA-Exportelements erfolgt.
24. DNA-Sequenz nach Punkt 23,
   dadurch gekennzeichnet, dass
   dass sie weiterhin in operativer Verknüpfung mit einer Transkriptionskontrollsequenz steht.
25. DNA-Sequenz nach Punkt 24,
   dadurch gekennzeichnet, dass
   dass die Transkriptionskontrollsequenz einen konstitutiv aktiven Promotor umfasst.
26. DNA-Sequenz nach Punkt 25,
   dadurch gekennzeichnet, dass
   der konstitutiv aktive Promotor der CMV (Cytomegalovirus) oder der SV40 (Simian Virus 40) Promotor ist.
27. DNA-Sequenz nach Punkt 23,
   dadurch gekennzeichnet, dass
   die Transkriptionskontrollsequenz einen induzierbarer Promotor umfasst.
28. DNA-Sequenz nach Punkt 27,
   dadurch gekennzeichnet, dass
   der induzierbare Promotor ein Tetracyclin-abhängiger Promotor ist.
29. DNA-Sequenz nach einem der Punkte 22 bis 28,
   dadurch gekennzeichnet, dass
   sie weiterhin ein Polyadenylierungssignal enthält.
30. DNA-Sequenz nach einem der Punkte 22 bis 29,
   dadurch gekennzeichnet, dass
   das Reporterprotein aus einem fluoreszierenden Protein, einem Protein, dessen enzymatische Aktivität nachweisbar ist, einem immunologisch nachweisbaren Protein, einem Protein, das als Selektionsmarker dient oder einem Transkriptionsfaktor ausgewählt ist.
31. DNA-Sequenz nach einem der Punkte 22 bis 30,
   dadurch gekennzeichnet, dass
   das cis-aktive RNA-Exportelement aus MPMV CTE (Mason-Pfizer monkey virus constitutive transport element), RSV CTE (Rous sarkoma virus constitutive transport element), SRV CTE (simian retrovirus constitutive transport element) oder HIV-1 RRE (human immunodeficiency virus 1 Rev responsive element), HIV-2 RRE (human immunodeficiency virus 2 Rev responsive element), SIV RRE, (simian immunodeficiency virus Rev responsive element), HTLV-1 (Human T-cell leukemia virus 1) Rex responsive element oder HTLV-11 (Human T-cell leukemia virus 11) Rex responsive element ausgewählt ist.
32. Eukaryontische Zelle,
   dadurch gekennzeichnet, dass
   sie mit einem DNA-Konstrukt nach einem der Punkte 22 bis 31 transformiert ist, wobei das DNA-Konstrukt in einer transkriptionsfähigen Form vorliegt.
33. Eukaryontische Zelle nach Punkt 32,
   dadurch gekennzeichnet, dass
   sie einen oder mehrere heterologe RNA-Exportfaktoren exprimiert.
34. Eukaryontische Zelle nach Punkt 33,
   dadurch gekennzeichnet, dass
   es sich bei den RNA-Exportfaktoren um virale Proteine handelt.
35. Eukaryontische Zelle nach Punkt 34,
   dadurch gekennzeichnet, dass
   der RNA-Exportfaktor ausgewählt ist aus Rev von HIV-1 (human immunodeficiency virus 1), HIV-2 (human immunodeficiency virus 2), SIV (simian immunodeficiency virus), EIAV (equine infectious anemia virus), Rex von HTLV-1 (human T-cell leukemia virus 1) oder HTLV-II (human T-cell leukemia virus II), oder den 34K und E4orf6 Proteinen von Adenoviren, oder EB2 Proteinen von EBV (Epstein-Barr virus), oder ORF 57 Genprodukten von Herpesvirus Saimiri, oder ICP 27 Proteinen von HSV (Herpes simplex virus).
36. Eukaryontische Zelle nach einem der Punkte 32 bis 35,
   dadurch gekennzeichnet, dass
   sie transformiert ist mit mindestens einer Nukleinsäure, die für ein weiteres Reporterproteins kodiert, das sich in Gegenwart des Reporterproteins für den RNA-Export nachweisen lässt.
37. Reagenzienkit, enthaltend
   (a) eine DNA-Sequenz nach einem der Punkte 23 bis 31 und eine mit der DNA-Sequenz transfizierbare Zelle oder/und
   (b) eine Zelle nach einem der Punkte 32 bis 36.
38. Reagenzienkit nach Punkt 37, weiterhin enthaltend Leptomycin B.
39. Verfahren zum Nachweis einer Virusinfektion,
   dadurch gekennzeichnet, dass
   die Aktivität eines viralen Kernexportfaktors bestimmt wird.
40. Verfahren nach Punkt 39,
   dadurch gekennzeichnet, dass
   es sich bei der Virusinfektion um eine Infektion mit einem Retrovirus handelt.
41. Verfahren nach Punkt 39,
   dadurch gekennzeichnet, dass
   es sich bei der Virusinfektion um eine Infektion mit einem Lentivirus handelt.
42. Verfahren nach Punkt 41,
   dadurch gekennzeichnet, dass
   es sich bei der Virusinfektion um eine Infektion mit HIV-1 (human immunodeficiency virus 1) oder HIV-2 (human immunodeficiency virus 2) handelt.
43. Verfahren nach Punkt 42,
   dadurch gekennzeichnet, dass
   der Rev-abhängige RNA-Export nachgewiesen wird.
44. Verfahren nach einem der Punkte 39 bis 43 zum Nachweis von Viren in einer Plasmaprobe.
45. Verfahren nach Punkt 44,
   dadurch gekennzeichnet, dass
   eine eukaryontische Zelle nach Punkt 32 verwendet wird.
46. DNA-Sequenz, die für ein Fluoreszenzprotein kodiert,
   dadurch gekennzeichnet, dass
   die für das Fluoreszenzprotein kodierende DNA-Sequenz gegenüber der Wildtypsequenz auf Nukleinsäureebene derart modifiziert ist, dass die Codonauswahl zumindest teilweise an die bevorzugte Codonauswahl eines Retrovirus, insbesondere eines humanen Retrovirus wie HIV-1 angepasst ist.
47. DNA-Sequenz nach Punkt 46,
   dadurch gekennzeichnet, dass
   sie die in Abb. 1 gezeigte hivGFP Sequenz umfasst.
48. DNA-Sequenz, umfassend eine für ein fluoreszierendes Protein kodierende Gensequenz zwischen einem funktionalen 5'-Spleißdonor und einem funktionalen 3'-Spleißakzeptor.
49. Verwendung der DNA-Sequenz nach Punkt 48 zur Untersuchung von RNA-Exportvorgängen.
50. Verwendung der DNA-Sequenz nach Punkt 48 zur Identifikation von Modulatoren des RNA-Exports aus dem Zellkern eukaryontischer Zellen.
51. Verfahren zum Nachweis des RNA-Exports aus dem Zellkern eukaryontischer Zellen, dadurch gekennzeichnet, dass ein Reporterprotein verwendet wird, dass ohne Lyse oder Fixierung der Zellen nachgewiesen werden kann.
52. Verfahren nach Punkt 51,
   dadurch gekennzeichnet, dass
   als Reporterprotein ein fluoreszentes Protein verwendet wird.
53. Verfahren nach Punkt 51,
   dadurch gekennzeichnet, dass
   als Reporterprotein ein Selektionsmarker verwendet wird.
54. Verfahren nach einem der Punkte 51 bis 53, dadurch gekennzeichnet, dass
   (a) die Zellen mit einer Genbibliothek transfiziert oder infiziert werden
   (b) die Gene, die eine Modulation des RNA-Exports aus dem Zellkern bewirken, durch Kultivieren der Zellen und Isolation der Nukleinsäure aufgereinigt werden.

## Patentansprüche

1. DNA-Sequenz, die für ein Fluoreszenzprotein kodiert,
**dadurch gekennzeichnet,**
**dass** die für das Fluoreszenzprotein kodierende DNA-Sequenz gegenüber der Wildtypsequenz auf Nukleinsäureebene derart modifiziert ist, dass die Codonauswahl zumindest teilweise an die bevorzugte Codonauswahl eines Retrovirus angepasst ist.

2. DNA-Sequenz nach Anspruch 1,
**dadurch gekennzeichnet,**
**dass** sie die in Abb. 1 gezeigte hivGFP Sequenz umfasst.

3. DNA-Sequenz nach Anspruch 1, umfassend eine für ein fluoreszierendes Protein kodierende Gensequenz zwischen einem funktionalen 5'-Spleißdonor und einem funktionalen 3'-Spleißakzeptor.

4. Verwendung der DNA-Sequenz nach Anspruch 3 zur Untersuchung von RNA-Exportvorgängen oder/und zur Identifikation von Modulatoren des RNA-Exports aus dem Zellkern eukaryontischer Zellen.
